# FASCICULE DE BREVET EUROPEEN

(11) **EP 0 493 581 B1**
(45) Date de publication et mention de la délivrance du brevet: **19.12.2001**
(21) Numéro de dépôt: 91913985.7
(22) Date de dépôt: 24.07.1991
(51) Int. Cl.: C12N 15/56, C12N 15/82, C12N 9/24, C12N 5/10

(54) **GENE RECOMBINANT CODANT POUR UNE PROTEINE A ACTIVITE ENDOCHITINASE**
FÜR EIN PROTEIN MIT ENDOCHITINASE-AKTIVITÄT KODIERENDES REKOMBINANTES GEN
RECOMBINANT GENE CODING FOR A PROTEIN HAVING AN ENDOCHITINASE ACTIVITY

(30) Priorité: 24.07.1990 FR 9009460
(43) Date de publication de la demande: 08.07.1992
(73) Titulaire: Biogemma, 75001 Paris (FR)
(72) Inventeur: DUBOIS, Michel, F-78530 Buc (FR); GRISON, René, F-31750 Escalquens (FR); LEGUAY, Jean-Jacques, F-31320 Auzeville-Tolosane (FR); PIGNARD, Annie, F-31120 Roquettes (FR); TOPPAN, Alain, F-31700 Cornebarrieu (FR)
(74) Mandataire: Gillard, Marie-Louise
(86) Numéro de dépôt international: FR9100607
(87) Numéro de publication internationale: WO9201792

(56) Documents cités:
- EP-A- 0 292 435
- WO-A-90/07001
- PLANT MOL. BIOL., Volume 14, 1990, SHISHI H. et al., "Structure of a Tobacco Endochitinase Gene: Evidence That Different Chitinase Genes Can Arise by Transposition of Sequences Encoding a Cysteine-rich Domain", pages 357-368.
- PASCAL ABREGE, No. 88-0108006, DURAND-TARDIF M., "Etude de l'induction, par l'etephon, de l'expression du Gene Codant Pour la Chitinase Chez la Tomate et Analyse....".
- MOL. PLANT-MICROB. INTERACT., Volume 3, No. 4, 1990, LINTHORST H.J.M. et al., "Analysis of Acid and Basic Chitinases from Tobacco and Petunia and Their Constitutive Expression in Transgenic Tobacco", pages 252-258.

## Description

L'invention concerne un nouveau gène recombinant codant pour une nouvelle protéine à activité endochitinase ou pour un précurseur de cette dernière, une bactérie contenant ce gène recombinant, une cellule végétale, une plante, ou une partie de plante, en particulier une semence végétale, qui contiennent un gène recombinant de ce type, un procédé pour rendre les plantes résistantes aux agents pathogènes, tels que les champignons, les bactéries, ainsi que les arthropodes notamment les insectes, et les nématodes, qui comprend une étape de transformation par ce gène, ainsi que cette nouvelle protéine et un procédé pour la préparer.

Les plantes cultivées sont soumises à des attaques par des agents pathogènes, tels que les champignons et les bactéries, lesquelles sont responsables de pertes importantes de récoltes. Le principal moyen actuel de lutte contre ces agents réside dans l'utilisation de substances chimiques à activité fongicide ou bactéricide. On sait aujourd'hui que les plantes réagissent naturellement à ces attaques par divers mécanismes de défense, malheureusement en général à déclenchement trop tardif et d'intensité trop faible pour être suffisamment efficaces. L'un de ces mécanismes comprend l'induction d'une enzyme appelée chitinase EC 3.2.1.14. (A. Toppan et al., 1982, Agronomie, 2, 829-834). Cette induction peut être artificiellement stimulée par des substances, telles que l'éthylène avec comme conséquence l'augmentation de la résistance aux agents pathogènes de la plante traitée (Bolier T., 1988, Oxford Surveys of Plant Molecular and Cell Biology, 5, 145-174).

La chitine est un polymère linéaire polysaccharidique, constitué d'unités N-acétylglucosamine associées par des liaisons β-1,4. Celui-ci est un composé structural présent dans la paroi de la plupart des champignons pathogènes, dans l'exosquelette des arthropodes, en particulier des insectes, et dans l'enveloppe externe des oeufs et des cystes de nématodes. Les enzymes appelées chitinases sont capables de dégrader la chitine. On distingue parmi celles-ci deux groupes différents définis selon leur mode d'attaque de la chitine : les exochitinases capables de libérer l'unité N-acétylglucosamine située aux extrémités non réductrices des chaînes et les endochitinases capables de fragmenter les chaînes, qui sont les seules chitinases capables d'inhiber in vitro la croissance des hyphes mycéliennes (Roberts W.K. et al., 1988, Gen. Microbiol, 134, 169-176). Les chitinases végétales connues sont en grande majorité de type endo, contrairement aux chitinases bactériennes connues qui sont de type exo (Roberts W.K et al., 1988, Gen. Microbiol, 134, 169-176).

Un grand nombre d'endochitinases végétales, notamment celles de tomate et de tabac (P. AUDY et al., 1990, Phytochem, 29, 4, 1143-1159), présentent également une activité lysozyme, capacité de cliver les liaisons β-1,4 entre la N-acétylglucosamine et l'acide N-acétylmuramique du peptidoglycane des parois bactériennes. On peut donc admettre que les activités lysozyme et endochitinase sont assez proches (Roberts W.K et al., 1988, Gen. Microbiol., 134, 169-176) et qu'une nouvelle protéine à activité endochitinase, en particulier de structure intermédiaire entre l'endochitinase de tomate et l'endochitinase de tabac, présente probablement une activité lysozyme.

Des séquences d'ADN codant pour des exochitinases bactériennes ont déjà été isolées et clonées (Jones J.D.G. et al., 1986 EMBO J., 5, 467-473 et Sundheim L. et al., 1988, Physiol. Molec. Plant Pathol., 33, 483-491). Le brevet US-A-4751081 décrit l'isolement et le clonage du gène complet codant pour la chitinase de Serratia marcescens ainsi que la transformation par ce gène des bactéries Pseudomonas fluorescens NZ130 et Pseudomonas putida MK280. Ces bactéries transformées sont capables de dégrader légèrement une chitine colloîdale dispersée dans le milieu de culture bactérien. Les travaux de Harpster M.H. et al, 1989, Nucl. Ac. Res., 17, 5395 ont montré que ce gène code pour une exochitinase, ce qui explique la faible efficacité de dégradation observée (Cf. tableau 2, col. 13 et 14 de ce document). La publication de Jones J.D.G. et al. (1988), Mol. Gen. Genet., 212, 536-542, mentionne la transformation de plantes de tabac par Agrobacterium tumefaciens contenant un gène chimérique comprenant la partie codante de l'exochitinase de Serratia marcescens, sous contrôle de différents promoteurs végétaux. Ce document ne donne aucune indication sur l'éventuelle augmentation de la résistance aux pathogènes conférée par l'expression de cette exochitinase.

Des séquences d'ADN génomique et/ou d'ADN complémentaire codant pour certaines endochitinases végétales ont d'autre part été isolées et clonées (Broglie K.E., 1986, Proc. Ntl. Acad. Sci. USA, 83, 6820-6824 et Hedrick S.A., 1988, Plant Physiol., 86, 182-186).

La demande de brevet WO 90/07001 décrit la construction d'un plasmide portant un ADN complémentaire de l'endochitinase de haricot Phaseolus vulgaris sous le contrôle d'un promoteur fort, la transformation à l'aide d'Agrobacterium tumefaciens, la régénération du tabac transformé, des essais montrant la résistance accrue aux champignons, Rhizoctonia solani et Botrvtis cinerea des plantes régénérées, l'obtention de plantes de tomates transgéniques exprimant la chitinase de haricot ainsi que l'obtention à l'aide de ce gène de plantes de colza transgéniques ayant un niveau d'activité chitinase et une résistance à Rhizoctonia solani accrues par rapport aux plantes de colza non transformées.

L'invention concerne donc un nouveau gène recombinant caractérisé en ce qu'il code pour une protéine à activité endochitinase ou un précurseur de cette dernière, qui comprend la séquence (1) ci-après **(SEQ ID NO : 1) :**

Gly Gly Asp Leu Gly Ser Val Ile Ser Asn Ser Met Phe Asp Gln Met Leu
Lys His Arg Asn Glu Asn Ser Cys Gln Gly Lys Asn Asn Phe Tyr Ser Tyr
Asn Ala Phe Ile Thr Ala Ala Arg Ser Phe Pro Gly Phe Gly Thr Ser Gly
Asp Ile Asn Ala Arg Lys Arg Glu Ile Ala Ala Phe Phe Ala Gln Thr Ser
His Glu Thr Thr Gly Gly Trp Pro Ser Ala Pro Asp Gly Pro Phe Ala Trp
Gly Tyr Cys Phe Leu Arg Glu Arg Gly Asn Pro Gly Asp Tyr Cys Ser Pro
Ser Ser Gln Trp Pro Cys Ala Pro Gly Arg Lys Tyr Phe Gly Arg Gly Pro
Ile Gln Ile Ser His Asn Tyr Asn Tyr Gly Pro Cys Gly Arg Ala Ile Gly
Val Asp Leu Leu Asn Asn Pro Asp Leu Val Ala Thr Asp Pro Val Ile Ser
Phe Lys Thr Ala Ile Trp Phe Trp Met Thr Pro Gln Ser Pro Lys Pro Ser
Cys His Asp Val Ile Ile Gly Arg Trp Asn Pro Ser Ala Gly Asp Arg Ser
Ala Asn Arg Leu Pro Gly Phe Gly Val Ile Thr Asn Ile Ile Asn Gly Gly

Leu Glu Cys Gly Arg Gly Asn Asp Asn Arg Val Gln Asp Arg Ile Gly Phe
Tyr Arg Arg Tyr Cys Gly Ile Leu Gly Val Ser Pro Gly Asp Asn Leu Asp
Cys Gly Asn Gln Arg Ser Phe Gly Asn Gly Leu Leu Val Asp Thr Met

Ce gène recombinant code de préférence pour une protéine qui comprend, immédiatement en amont de la séquence (1), la séquence (2) ci-après **(SEQ ID NO : 2) :** 
Gln Asn Cys Gly Ser Gln Gly Gly Gly Lys Val Cys Ala Ser Gly Gln Cys
Cys Ser Lys Phe Gly Trp Cys Gly Asn Thr Asn Asp His Cys Gly Ser Gly
Asn Cys Gln Ser Gln Cys Pro Gly Gly Gly Pro Gly Pro Gly Pro Val Thr
 destinée à être clivée lors de la maturation de la protéine, ou une séquence présentant un degré d'homologie substantiel avec la séquence (2).

De préférence, ce gêne recombinant code pour une protéine dont la séquence comprend, en amont de la séquence (1) et de préférence séparée de la séquence (1) par une séquence destinée à être clivée, une séquence codant pour un peptide-signal. Un gène de ce type particulièrement intéressant est celui qui code pour une protéine dont la séquence comprend, immédiatement en amont de la séquence (2) ou d'une séquence présentant un degré d'homologie substantiel avec la séquence (2), la séquence (3) ci-après **(SEQ ID NO : 3) :** 
Met Arg Arg Thr Ser Lys leu Thr Thr Phe Ser Leu Leu Phe Ser Leu Val
Leu Leu Ser Ala Ala Leu Ala
 ou une séquence présentant un degré d'homologie substantiel avec la séquence (3).

L'invention concerne aussi un gène recombinant codant pour une protéine à activité endochitinase ou pour un précurseur de cette dernière, qui comprend une séquence présentant un degré d'homologie substantiel avec la séquence (1). La partie codante de ce gène recombinant comprend au moins une partie 5' de l'ADN génomique ou de l'ADN complémentaire de l'endochitinase de tomate et au moins une partie 3' de l'ADN génomique ou de l'ADN complémentaire de l'endochitinase de tabac. De préférence, la partie codante du gène recombinant présente au moins un intron. En effet, il est connu que la présence d'introns dans la partie codante d'un gène augmente l'expression de celui-ci (voir par exemple les travaux de J. CALLIS et al., (1987, Genes and devetopment, 1, 1183-1200).

Un exemple de tel gène recombinant est le gène recombinant dont la partie codante est la séquence ci-après **(SEQ ID NO : 4) :**

ATGAGGCGAA CTTCTAAATT GACTACTTTT TCTTTGCTGT TTTCTCTGGT
TTTGCTGAGT GCTGCCTTGG CACAGAATTC TGGTTCACAG GGCGGAGGCA
AAGTTTGTGC GTCGGGACAA TGTTGCAGCA AATTCGGGTG GTGCGGTAAC
ACTAATGACC ATTGTGGTTC TGGCAATTGT CAAAGTCAGT GTCCAGGTGG
CGGCCCTGGT CCTGGTCCTG TTACTGGTGG GGACCTCGGA AGCGTCATCT
CAAATTCTAT GTTTGATCAA ATGCTTAAGC ATCGTAACGA AAATTCTTGT
CAAGGAAAGA ATAATTTCTA CAGTTACAAT GCCTTTATTA CTGCTGCTAG
GTCTTTTCCT GGCTTTGGTA CAAGTGGTGA TATCAATGCC CGTAAAAGGG
AAATTGCTGC TTTCTTTGCC CAAACCTCCC ATGAAACTAC TGGTATGTGT
ATAACCATTC ACATCGAACC ATTAAAATAT AATTTCATTT TATTTTATTT
AGTAATTGAT TATATATGTA GGAGGATGGC CTTCCGCACC TGATGGACCA
TTCGCATGGG GTTACTGTTT CCTTAGAGAA CGAGGTAACC CCGGTGACTA
CTGTTCACCA AGTAGTCAAT GGCCTTGTGC ACCTGGAAGG AAATATTTCG
GACGAGGCCC AATCCAAATT TCACAGTAAG CTACATAAAT CTATATATGG
TAAAATTTGA TGAACTTGTA GTGTCTAATT ACGTGTATTT TGACATTTCA
AAACAGCAAC TACAACTATG GGCCATGTGG AAGAGCCATC GGAGTGGACC
TTTTAAACAA TCCTGATTTA GTAGCCACAG ACCCAGTCAT CTCATTCAAG
ACTGCTATCT GGTTCTGGAT GACCCCTCAA TCACCAAAGC CTTCTTGCCA
CGATGTCATC ATTGGAAGAT GGAACCCATC TGCCGGTGAC CGATCAGCCA

ATCGTCTTCC TGGATTTGGT GTCATCACAA ACATCATCAA TGGGGGCCTG
GAATGTGGTC GTGGCAATGA CAATAGGGTC CAGGATCGCA TTGGGTTTTA
CAGGAGGTAT TGCGGTATTC TTGGTGTTAG TCCTGGTGAC AATCTTGATT
GCGGAAACCA GAGATCTTTT GGAAACGGAC TTTTAGTCGA TACTATGTAA
TGA

Cette séquence codante est, de préférence, précédée d'une séquence promotrice comportant un promoteur viral fort, tel que le promoteur 35S du virus de la mosaïque de chou-fleur [voir ODELL J.T. et al., 1985, NATURE, 313, 810-812] et suivie d'une séquence terminatrice comportant le terminateur de la nopaline synthase d'Agrobacterium tumefaciens [voir BEVAN M. et al., 1983, Nucl. Ac. Res., 11, 369].

L'invention a également trait à une bactérie, par exemple de l'espèce E. coli, qui contient le gène recombinant défini ci-dessus dans un contexte ou environnement nucléotidique permettant sa réplication et donc peut servir au clonage de ce gène, ainsi qu'à une bactérie susceptible d'infecter une plante avec transfert de matériel génétique, par exemple de l'une des espèces Agrobacterium rhizogenes et Agrobacterium tumefaciens, qui contient ce gène dans un contexte permettant sa réplication et donc peut servir à transformer des cellules végétales. La transformation des cellules végétales par le gène ci-dessus peut également être effectuée par une autre méthode biologique telle que la voie du tube pollinique (Zhong-xun Luo et al., Plant Molec. Biol. Rep., 1988, 6, 165-176) et la transformation directe de graines en germination (Toepfer R. et al., 1989, The Plant Cell., 1, 133-139), ou par une méthode physique telle que l'utilisation de polyéthylèncglycol, l'électroporation (Chistou P. et al., 1987, Proc. Ntl. Acad. Sci. USA, 84, 3662-3699) et le bombardement à l'aide de microprojectiles (Klein T.M. et al., 1988, Proc. Ntl. Acad. Sci. USA, 85, 8502-8505).

L'invention concerne également une cellule végétale caractérisée en ce qu'elle est transformée par le gène recombinant défini ci-dessus, inséré dans un contexte susceptible de permettre son expression. Cette cellule végétale peut provenir d'une espèce de grande culture, telle que par exemple le maïs, le soja, la betterave, le blé, l'orge, le pavot, le colza, le tournesol, la luzerne et le sorgho, d'une espèce florale telle que le rosier, l'oeillet, le gerbera ou d'une espèce potagère telle que la carotte, la tomate, la salade, la chicorée, le poivron, le melon et le chou. Des espèces particulièrement appréciées sont le colza Brassica napus, le tournesol Helianthus annuus et le tabac Nicotiana tabacum.

L'étape de transformation qui concerne une ou quelques cellules est suivie d'une étape de multiplication de ces cellules transformées de façon à obtenir des cals, lesquels peuvent donner naissance à des plantes transformées, par des processus d'organogenèse ou d'embryogenèse. Une partie des descendances de ces plantes transformées contient et exprime le gène recombinant.

L'invention concerne donc aussi une plante ou une partie de plante, caractérisée en ce qu'elle contient, dans un contexte susceptible de permettre son expression, le gène recombinant défini ci-dessus. Une partie de plante particulièrement appréciée est la semence : graine ou autre partie d'un végétal apte à former une nouvelle plante complète, notamment après semi ou enfouissement. Ces plantes peuvent être l'une quelconque des espèces ci-dessus et plus particulièrement des espèces Nicotiana tabacum, Helianthus annuus et Brassica napus.

L'invention a également trait à un procédé pour obtenir des plantes résistantes aux agents pathogènes, tels que les champignons, les bactéries ainsi que les arthropodes, en particulier les insectes, et les nématodes, caractérisé en ce qu'il comprend une étape de transformation de cellules végétales par le gène recombinant défini ci-dessus, suivie d'une étape de multiplication des cellules transformées et d'une étape de régénération des plantes.

De préférence, l'étape de transformation des cellules végétales est effectuée in vitro à l'aide d'une agrobactérie (c'est-à-dire d'une bactérie du genre Agrobacterium) ayant intégré le gène recombinant défini ci-dessus.

L'invention concerne aussi les plantes résistantes aux agents pathogènes susceptibles d'être obtenues à l'aide du procédé défini ci-dessus.

L'invention a également trait à l'utilisation d'une plante faisant partie des plantes définies au paragraphe précédent ou d'une plante contenant, dans un contexte susceptible de permettre son expression, le gène recombinant défini précédemment, comme géniteur dans un programme de sélection pour créer de nouvelles variétés végétales.

L'invention concerne aussi une nouvelle protéine à activité endochitinase qui comprend la séquence (1) ainsi qu'un procédé pour l'obtenir qui comprend la mise en culture de cellules ou de cals végétaux transformés par le gène recombinant, la lyse de ceux-ci, l'isolement et la purification de la protéine recombinante. Cette protéine peut présenter un intérêt comme principe actif d'un nouveau médicament destiné à soigner des affections, telles que par exemple les mycoses.

L'invention sera mieux comprise à l'aide des exemples ci-après :
une grande partie de l'ensemble des techniques ci-après, bien connues de l'homme de l'art, est exposée en détail dans l'ouvrage de Maniatis et al. : "Molecular cloning : a laboratory manual" publié en 1989 par les éditions Cold Spring Harbor Press à New York (2^{e} édition).

Le matériel biologique (souches, phages, plasmides ou plantes) utilisé dans les exemples ci-après est disponible dans le commerce et décrit respectivement dans les documents ci-après :
- phage Lambda CHARON 4A : MANIATIS et al., Op. Cit. ;
- vecteur binaire pBIN19 : BEVAN et al., 1984, Nucl. Ac. Res., 12, 8711-8721 ;
- plasmide pBI121 : JEFFERSON R.A. et al., 1987, E.M.B.O.J., 6, 3901 ;
- souche E. coli MC1061 : MEISSNER P.S. et al., 1987, Proc. Natl. Acad. Sci. USA, 84 4171 ;
- souche E. coli HB101 : MANIATIS et al., Op. Cit. ;
- souche Agrobacterium tumefaciens LBA4404 :
   HOEKEMA et al., 1983,
   NATURE, 303, 179-180 ;
- plante Nicotiana tabacum var. Wisconsin Havana 38 : SCHNEIDER M., 1990, Plant Molec. Biol., 14, 935-947 ;
- champignon Chalara elegans : RAWLINGS R.E., 1940, Ann. Mo. Bot. Gdn., 27, 561-598 ;
- plante Nicotiana tabacum var Paraguay 49 obtenue auprès de
   l'Institut du tabac, Bergerac, France.
- champignon Alternaria brassicae : BAINS et TEWARI, 1987, Physiol. Mol. Plant. Pathol. 30 : 259 ;
- plante Helianthus Annuus, variété Euroflor de Rustica Semences ;
- Sinapis alba : BAINS et TEWARI, référence ci-dessus.

Les abréviations suivantes sont utilisées dans les exemples ci-après :
- 32-dCTP :: déoxycytidine 5'-|§32-P£-triphosphate commercialisé par AMERSHAM sous la référence 10205 ;
- 0,2 x SSC :: NaCl 30 mM, citrate trisodique 3 mM ; pH 7,0 (décrit par MANIATIS et al., op. cit.) ;
- SDS :: dodécylsulfate de sodium ;
- FPLC :: fast protein liquid chromatography ;
- PVDF :: difluorure de polyvinylidène.

La description ci-après sera mieux comprise à l'aide des figures 1 à 6.

La figure 1 représente une carte de restriction du fragment d'ADN génomique de l'endochitinase de tomate d'environ 3,5 kb, inséré dans le plasmide p CH3,5.

La figure 2 représente la séquence d'ADN génomique, et la séquence peptidique déduite, de l'endochitinase de tomate de 3,5 kb, inséré dans le plasmide p CH3,5.

La figure 3 représente l'alignement d'après l'homologie maximale de l'ADN génomique de l'endochitinase de tomate dépourvue des introns, (ligne inférieure), et de l'ADN complémentaire de l'endochitinase de tabac (ligne supérieure).

La figure 4 représente la séquence codante du gène chimérique, encadrée par les sites Bam HI et Sac I, et la séquence d'acides aminés déduites.

La figure 5 représente la séquence du gène chimérique complet.

La figure 6 représente la séquence de l'endochitinase recombinante mature.

### EXEMPLE 1 : Construction du vecteur binaire pBR1 contenant un gène recombinant tomate-tabac de l'endochitinase

### 1) Préparation de la séquence codante du gène recombinant

### a) Préparation de la partie 5' de la séquence codante du gène recombinant à partir de l'ADNg (ADN génomique) de l'endochitinase de tomate :

Un clone contenant l'ADNg de l'endochitinase de tomate a été obtenu de la façon suivante (Cf. Thèse de Doctorat ès Sciences spécialité : biologie moléculaire végétale, 1986, de M. DURANT-TARDIF - Université de Paris Sud) :

Une banque d'ADN génomique de tomate a été construite dans le phage Lambda Charon 4A par clonage de fragments issus de la digestion partielle par l'endonucléase EcoRI de l'ADN génomique de tomate Lycopersicon esculentum. La banque génomique ayant été amplifiée, 6,6 x 10⁵ clones ont été criblés après transfert de l'ADN phagique sur nitrocellulose par les techniques bien connues de l'homme de l'art (Maniatis et al., Molecular cloning : a laboratory manual, Cold Spring Harbor Laboratory, 1984), à l'aide d'une sonde d'ADNc codant pour une endochitinase de haricot (Broglie et al., 1986, PNAS, 83, 6820-6824).

Le clone, appelé clone 10.2 s'hybridant avec cette sonde, contient un fragment d'ADN génomique de tomate de 3,5 kb contenant une partie du gène de l'endochitinase de tomate. Ce fragment a ensuite été inséré dans le plasmide pEMBL8 (Dente et al. (1983) Nucl. Ac. Res., 11, 1645-1655) entre les sites EcoRI et HindIII. Le plasmide obtenu appelé pCH3.5 a été cloné dans E. coli. Ce plasmide a ensuite été extrait et purifié par la méthode de la lyse alcaline (BIRNBOIM et DOLY dans Maniatis et al., op. cit.).

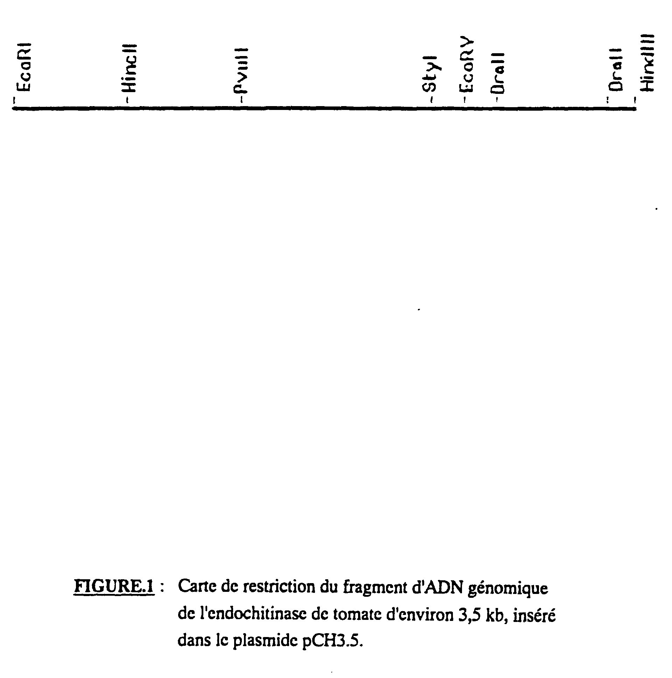

L'utilisation de plusieurs endonucléases de restriction a permis d'établir la carte de restriction du fragment d'ADN génomique d'environ 3,5 kb inséré dans le plasmide pCH3.5, représentée sur la figure 1.

Les différents fragments EcoRI-HincII, HincII-PvuII, PvuII-EcoRV, EcoRV-HindIII ont été préparés par digestion par les endonucléases correspondantes, purifiés par électrophorèse sur gel d'agarose et isolés par électroélution (Maniatis et al., op. cit.). Chacun de ces fragments a été cloné dans l'ADN de la forme réplicative du phage simple brin M13mp19 (Pharmacia) entre les sites de restriction compatibles. Ces fragments ont ensuite été séquencés selon la méthode des didéoxyribonucléotides (Sanger et al., PNAS-USA, 14, 5463-5467, 1977).

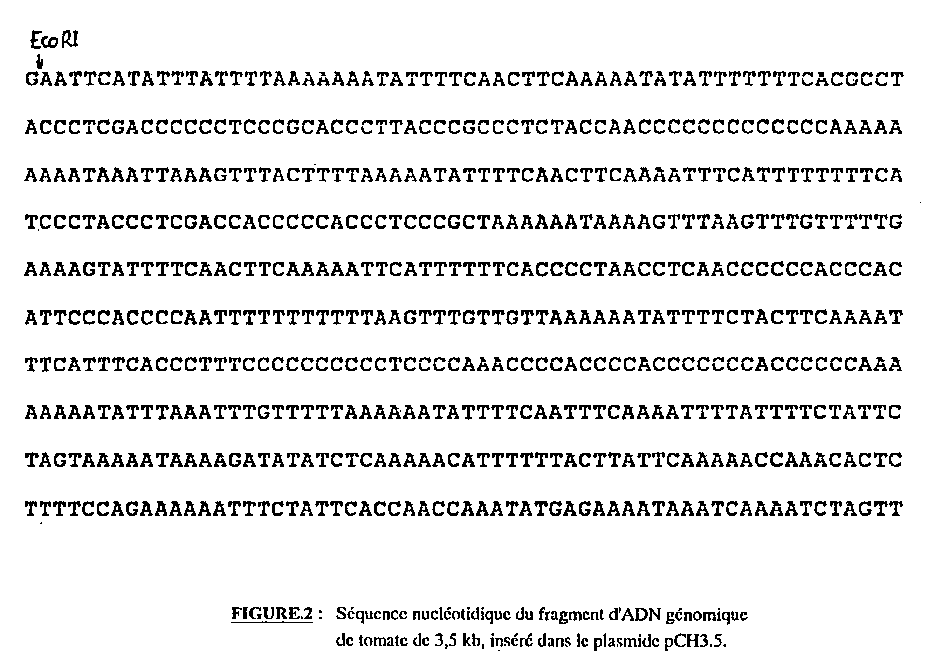

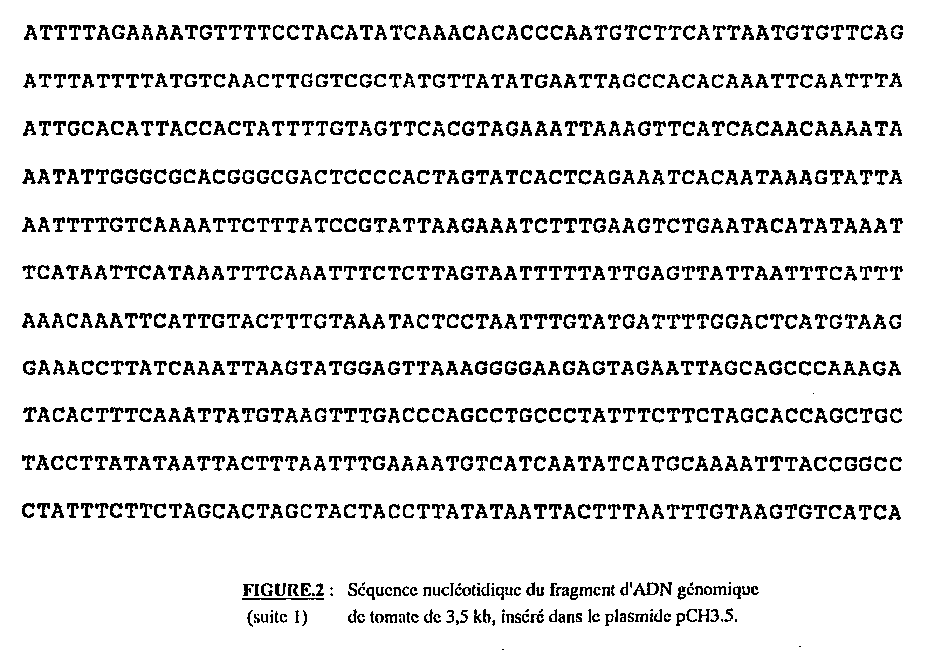

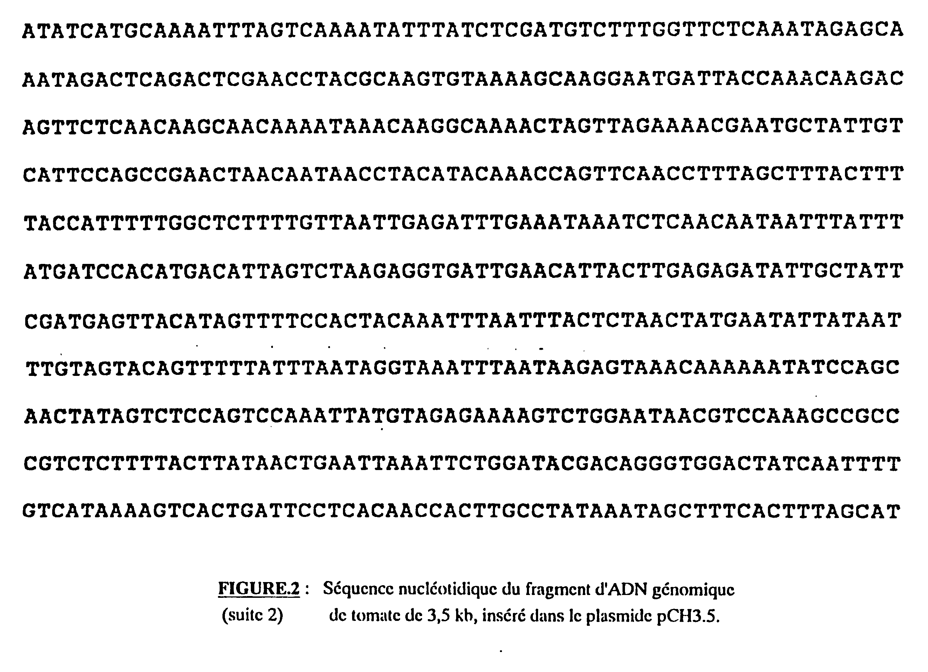

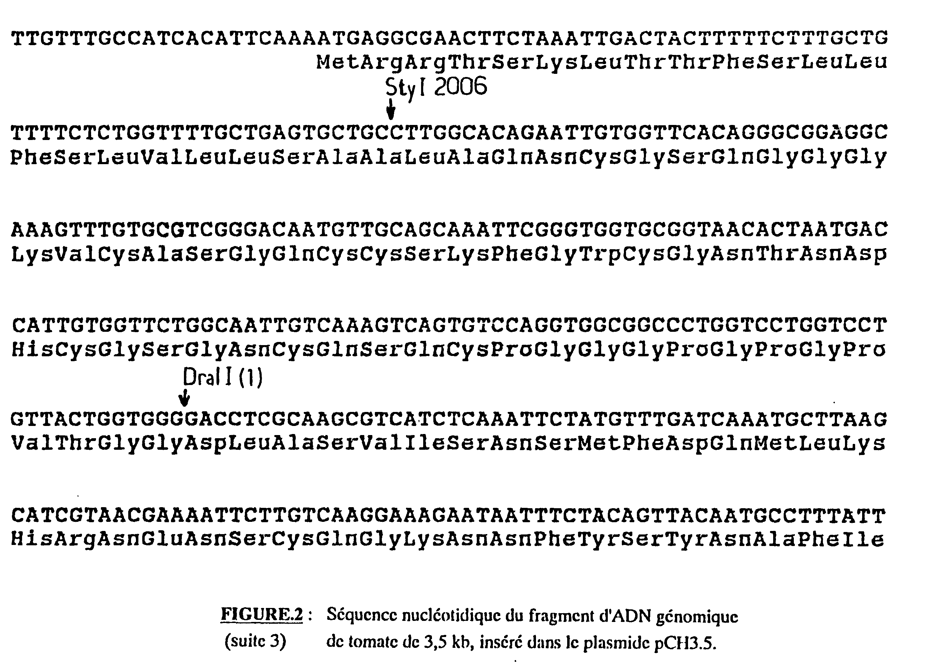

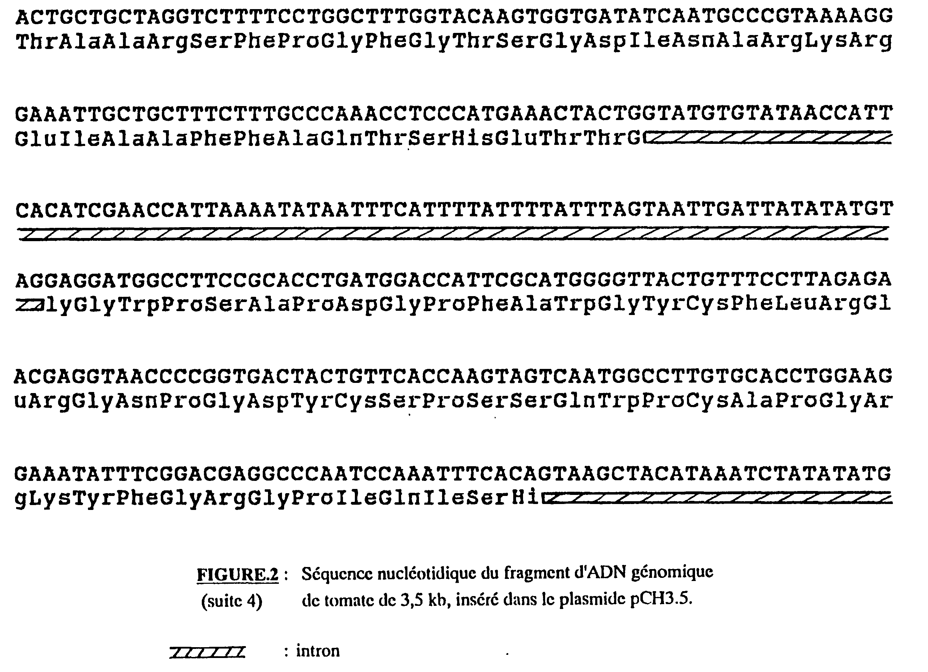

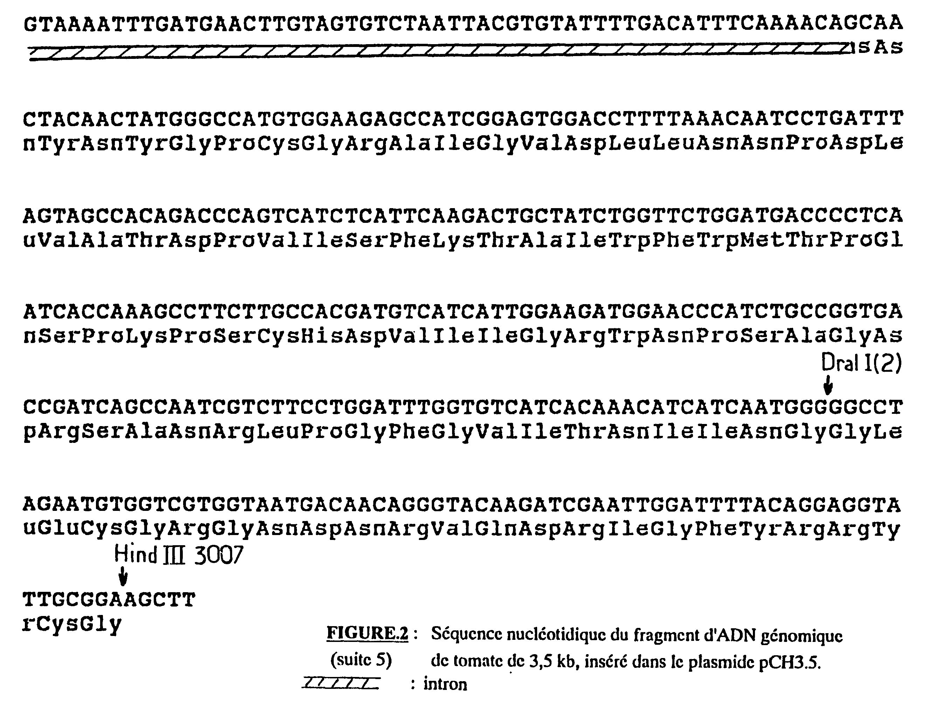

La séquence **(SEQ ID NO : 5),** telle que déduite des expériences ci-dessus, est représentée sur la figure 2, laquelle indique également les sites de restriction utilisés pour le clonage dans pEMBL8 et les sites de restriction importants pour la suite de la construction. La séquence d'acides aminés traduite est aussi représentée sur cette figure sur la ligne inférieure par rapport à la séquence codante, les in- ons étant hachurés (*IIIIII*).

Cette séquence possède une partie promotrice de 1940 nucléotides suivie d'une partie codante, codant pour 302 acides aminés, dans laquelle sont insérés deux introns. Cette partie codante est incomplète dans sa région 3' (pas de codon stop).

Par utilisation des sites de restriction StyI (position 2006) et HindIII (position 3007), un fragment de 1001 pb a été obtenu ; il a été purifié par électrophorèse sur gel d'agarose à bas point de fusion. La synthèse chimique d'un oligonucléotide de 71 pb, appelé fragment 1, dont la séquence est donnée ci-dessous, a permis de reconstituer la partie 5' éliminée en amont du site StyI et d'insérer en amont du codon ATG d'initiation de la traduction un site de restriction BamHI. Le fragment BamHI-HindIII de 1071 pb a été sous-cloné dans le vecteur pUC 19 (Pharmacia) dans les sites correspondants à l'aide de la T4 DNA ligase. Le plasmide obtenu est appelé pCH1.

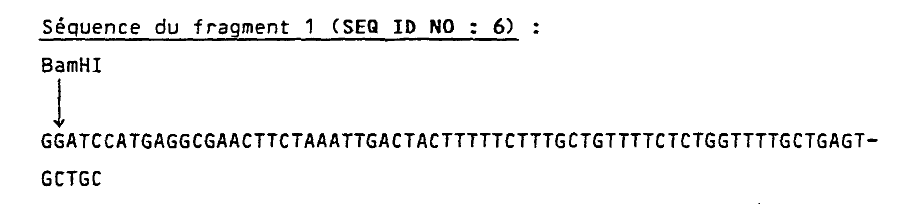
Séquence du fragment 1 (SEQ ID NO : 6) :
BamHI
GGATCCATGAGGCGAACTTCTAAATTGACTACTTTTTCTTTGCTGTTTTCTCTGGTTTTGCTGAGT-
GCTGC

### b) Préparation de la partie 3' manquante de la séquence codante du gène chimérique :

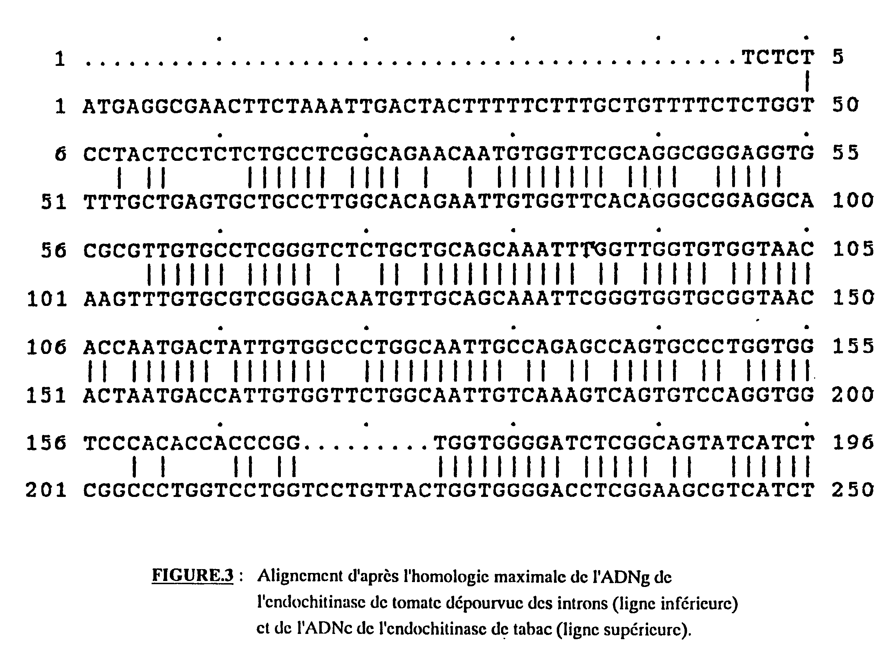

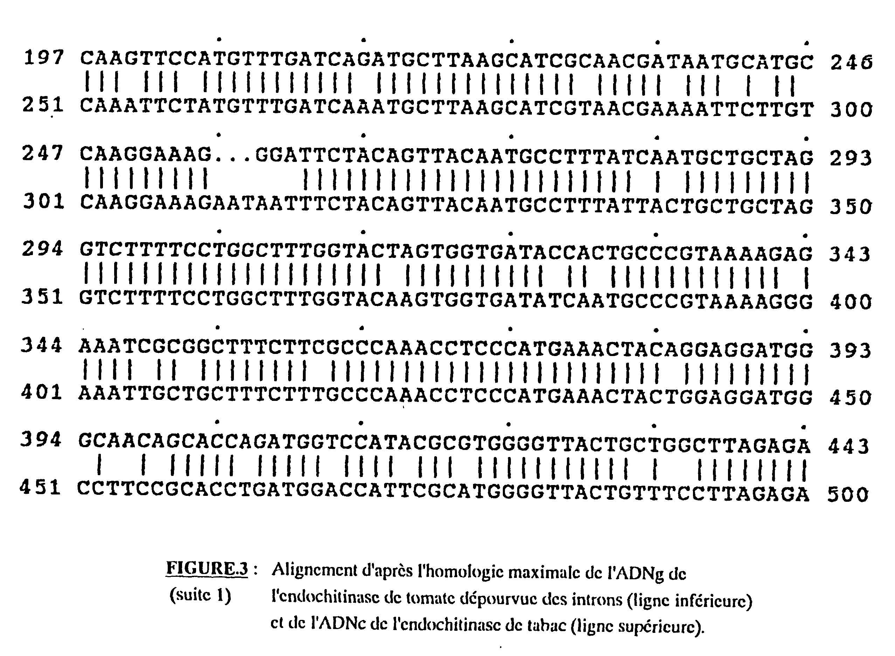

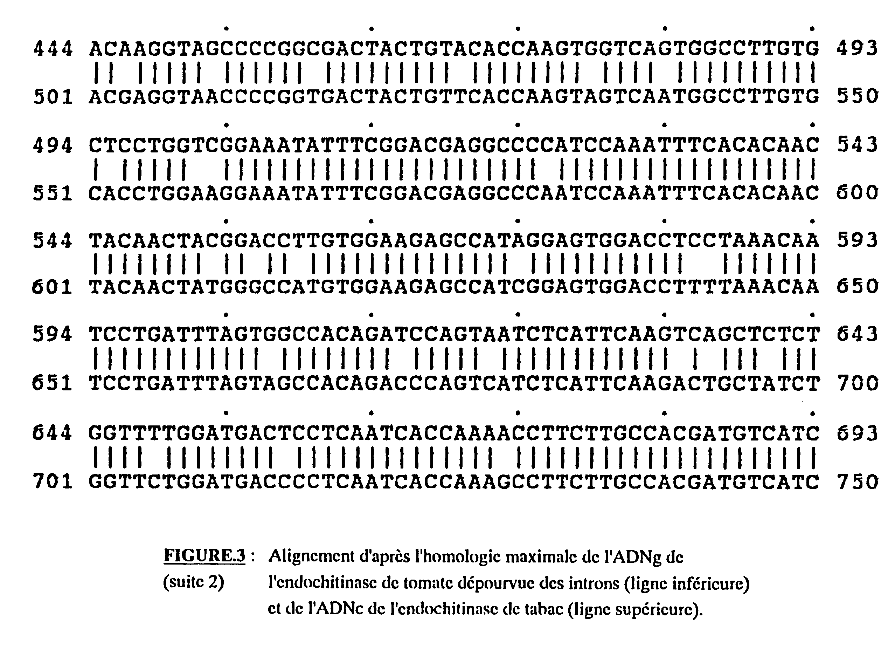

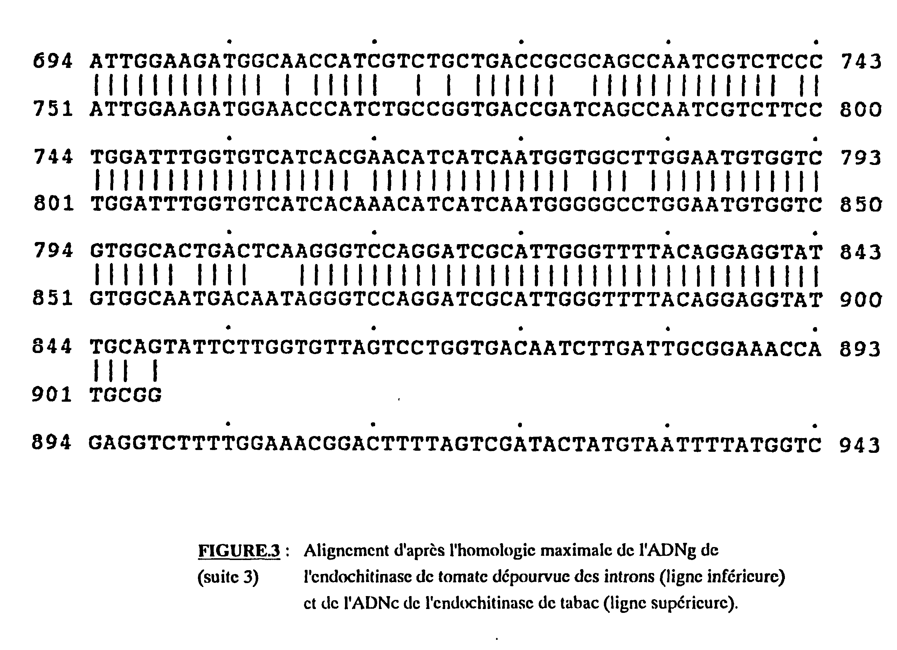

Une comparaison de séquences à l'aide d'un logiciel approprié (logiciel UWGCG de l'université de Wisconsin : Devereux et al., 1984, Nucl. Ac. Res., 12, 8711-8721 - Option GAP : alignement optimal des séquences d'après la méthode de Needleman et Wunsch, 1970 J. mol. Biol., 48, 443-453) entre la partie codante, incomplète dans sa région 3' du clone pCH3.5 et la séquence publiée de l'ADNc de l'endochitinase de tabac comprenant 329 acides aminés (Hideaki Shinshi et al. (1987), Proc. Ntl. Acad. Sci. USA, 84, 89-93 et (1990) Plant Mol. Biol., 14, 357-368) montre une homologie importante entre les séquences, en particulier dans la partie 3' de celles-ci (Cf. figure 3 qui représente un alignement effectué par ce logiciel, d'après l'homologie maximale de ces deux séquences, celle de l'ADNg de l'endochitinase de tomate dépourvue de ses introns étant située sur la Ligne inférieure).

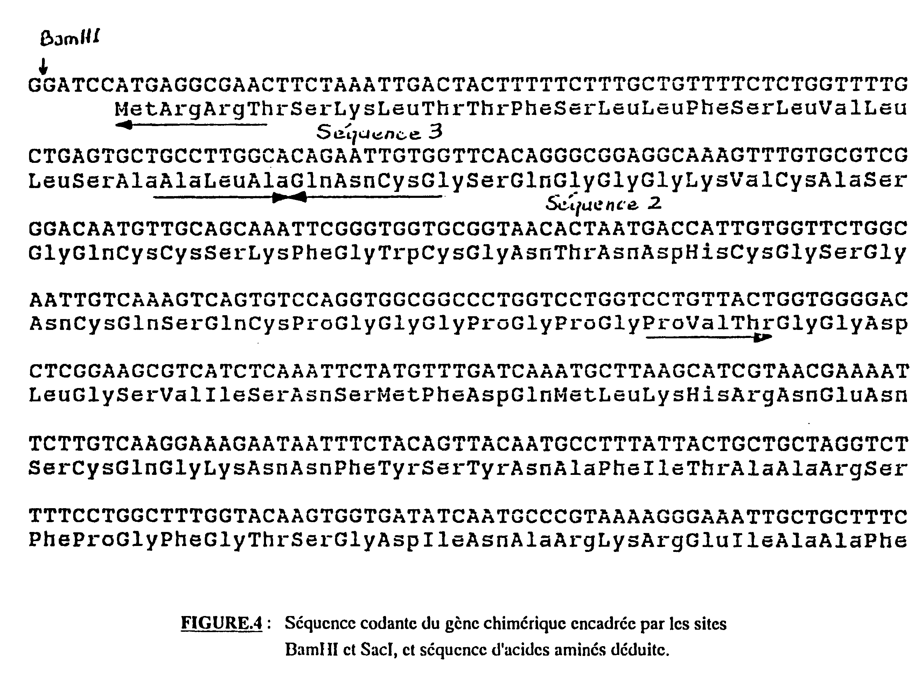

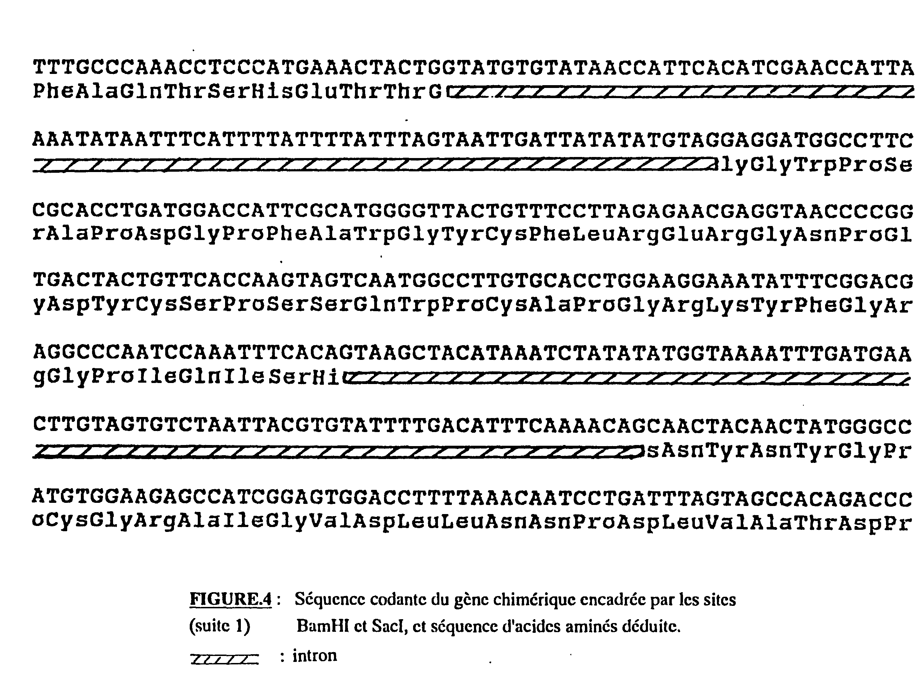

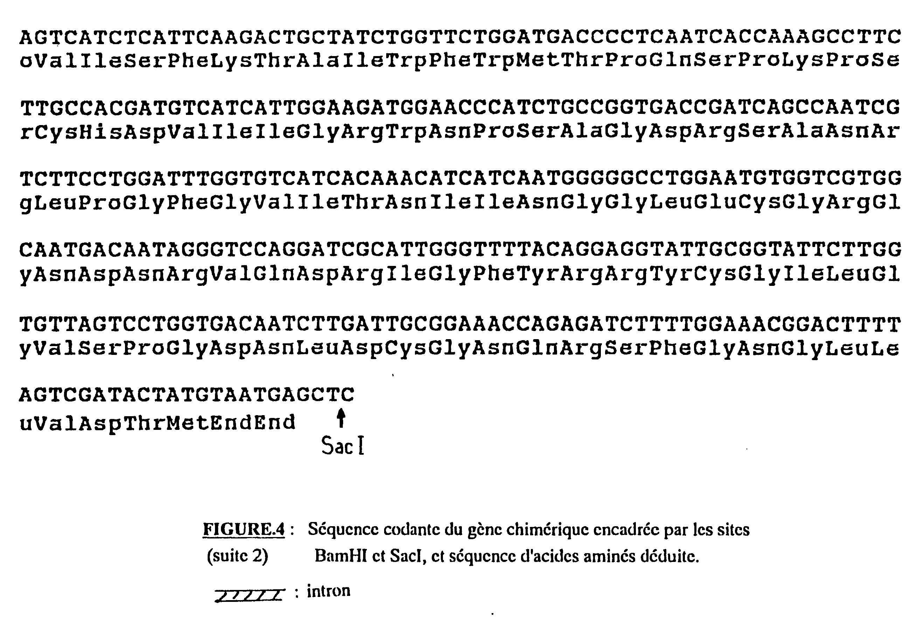

On a assemblé des oligonucléotides synthétisés sur un synthétiseur d'ADN Applied Biosystems 4600, de façon à obtenir un fragment, appelé fragment 2, dont la séquence reprend pour 71 nucléotides la séquence de l'ADNg de l'endochitinase de tomate, située en aval du site DraII (2) et pour 92 nucléotides, une séquence proche de la partie 3' de la séquence publiée de l'ADNc d'endochitinase de tabac, à laquelle on a rajouté un second codon stop et la séquence du site de restriction SacI. Cette séquence est représentée ci-après : la séquence proche de la partie 3' de la séquence de l'ADNc d'endochitinase de tabac étant soulignée et le site SacI étant indiqué **(SEQ ID NO : 7) :** 
GGCCTGGAA TGTGGTCGTG GCAATGACAA TAGGGTCCAG GATCGCATTG GGTTTACAG
GAGGTATTGC GGTATTCTTG GTGTTAGTCC TGGTGACAAT CTTGATTGCG GAAACCAGAG
ATCTTTTGGA AACGGACTTT TAGTCGATAC TATGTAATGA GCTC
↑
SacI
 On a soumis à une hydrolyse partielle par les enzymes de restriction BamHI et DraII le plasmide pCH1, puis isolé et purifié après électrophorèse sur gel d'agarose un fragment de 999 pb, appelé fragment 3, dont les extrémités sont constituées par le site BamHI en 5' et par le site DraII (2) en 3' (Cf. figure 2), les fragments 2 et 3 ont été ligués à l'aide de la T4 DNA ligase dans le plasmide pUC 19, ouvert aux sites de restriction BamHI et SacI. Le plasmide obtenu est appelé pCH1.2. On a vérifié par séquençage que la partie BamHI - SacI de ce plasmide comportait la séquence attendue. Celle-ci, ainsi que la séquence d'acides aminés déduite, est représentée sur la figure 4. Cette séquence comprend la séquence codante du gène chimérique encadrée par les sites de restriction BamHI et SacI. Celle-ci code pour une protéine de 329 acides aminés comprenant un peptide signal supposé de 24 acides aminés (déterminé à l'aide d'un logiciel qui utilise la méthode décrite par G. von Heijne, 1986, Nucl. Ac. Res., 14, 483-490). La masse moléculaire attendue d'après la séquence de cette protéine clivée de son peptide signal supposé est d'environ 32 kDa.

### 2) Préparation du gène chimérique complet et clonage de celui-ci dans le vecteur binaire pBIN 19.

La séquence codante obtenue précédemment a été insérée entre une séquence promotrice comprenant le promoteur dit 35S du virus de la mosaïque du chou-fleur (35S CaMV) et une séquence terminatrice comprenant le terminateur de la nopaline synthase (NOS) d'Agrobacterium tumefaciens.

### a) Préparation de la séquence promotrice comprenant le promoteur 35S du virus de la mosaïque du chou-fleur :

A partir du plasmide pBI121 (Clontech) par coupure à l'aide des endonucléases HindIII et BamHI, puis électrophorèse, le fragment HindIII-BamHI d'environ 900 pb contenant le promoteur 35S est isolé. Ce fragment est recoupé par HindII. Le fragment d'environ 410 pb, portant le site BamHI, est traité par la T4 DNA ligase en présence d'un linker HindIII (séquence synthétique contenant un site HindIII). Après coupure par l'endonucléase HindIII et électrophorèse, le fragment HindIII-BamHI résultant (d'environ 420 pb) est isolé et purifié.

### b) Préparation de la séquence terminatrice comprenant le terminateur de la nopaline synthase (NOS) d'Agrobacterium tumefaciens.

A partir du plasmide pBI121 (Clontech) par coupure à l'aide des enzymes de restriction SacI et EcoRI, puis électrophorèse sur gel d'agarose, un fragment de 250 pb environ, renfermant le terminateur de la nopaline synthase, a été isolé.

On a ligué à l'aide de la T4 DNA ligase la séquence promotrice, la séquence codante du gène chimérique de la chitinase et la séquence terminatrice dans le vecteur binaire pBIN19 ouvert à l'aide des endonucléases HindIII et EcoRI. La partie de ce vecteur qui pourra être transféré aux végétaux comprend un gène de résistance à la kanamycine immédiatement en amont du gène chimérique complet (Cf. BEVAN (1984), Nucl. Ac. Res., 12, 8711-8721). Le gène de résistance à la kanamycine servira de marqueur de sélection au cours des étapes de transformation et d'analyse de la descendance des plantes transformées.

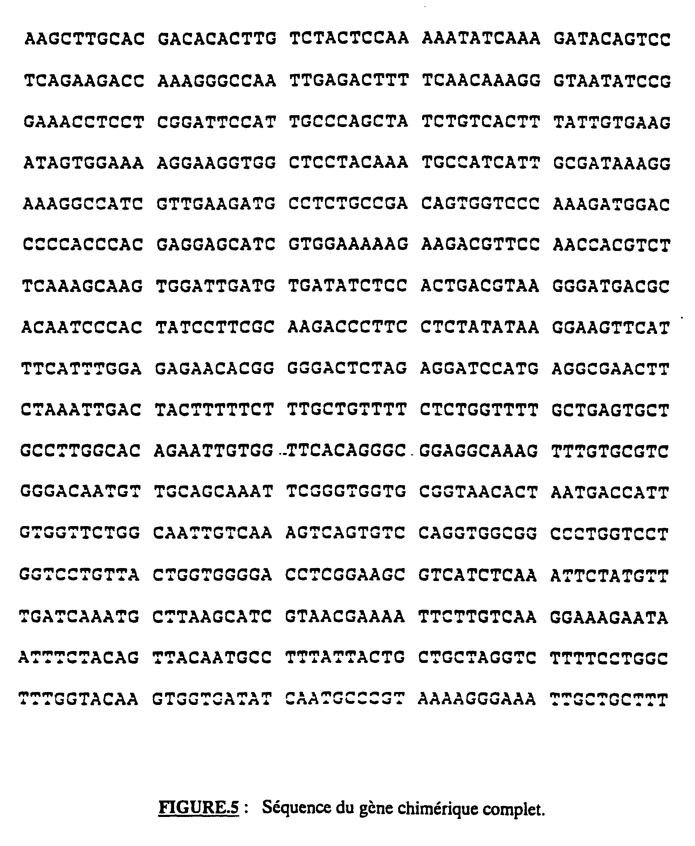

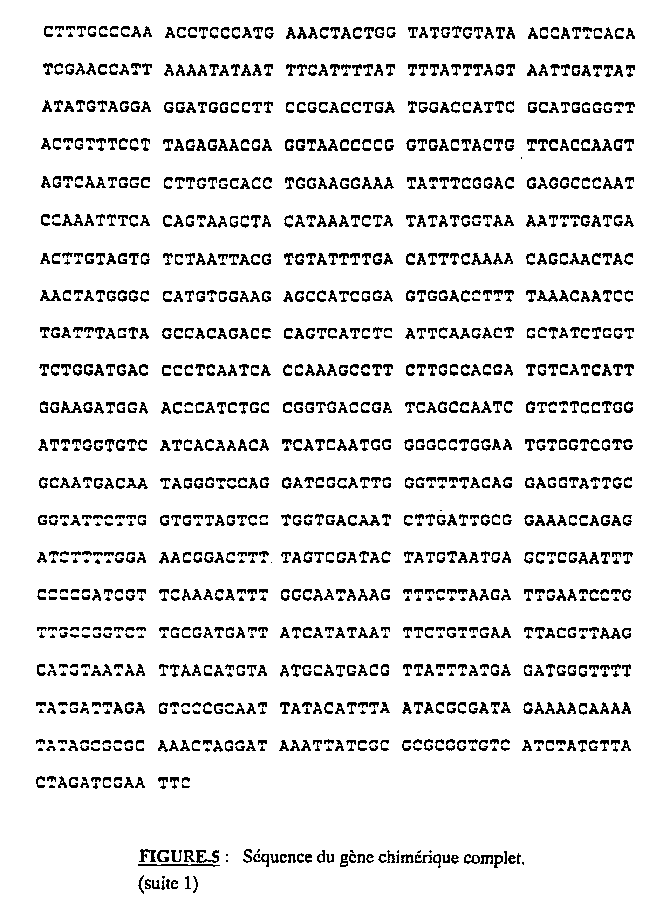

Le vecteur obtenu est appelé pBR1. La séquence du gène chimérique complet **(SEQ ID NO : 8),** vérifiée par séquençage, est représentée sur la figure 5. Le plasmide est cloné dans la souche E. coli MC1061 (Clontcch).

### EXEMPLE 2 : Transfert dans Agrobacterium tumefaciens ou Agrobacterium rhizogenes du plasmide pBR1 contenant le gène chimérique tomate-tabac de l'endochitinase.

### a) Transfert dans Agrobacterium tumefaciens

Ce transfert est réalisé comme décrit ci-après par conjugaison triparentale entre la souche E. coli MC1061 contenant le vecteur pBR1 et la souche Agrobacterium tumefaciens LBA4404 (Clontech) à l'aide de la souche E. coli HB101 contenant le plasmide mobilisateur pRK2013.

La souche E. coli MC1061 contenant le plasmide pBR1 et une souche E. coli HB101 (Clontech) contenant le plasmide mobilisateur pRK2013 sont cultivées à 37°C dans du milieu Luria (Gibco) en présence de 25 mg/l de kanamycine.

La souche Agrobacterium tumefaciens LBA4404 est cultivée à 28°C dans du milieu Luria en présence de 100 mg/l de rifampicine (elle est résistante à cet antibiotique) ; 200 µl de chacune des trois cultures sont mélangés, étalés sur du milieu Luria gélosé (Gibco) et incubés toute la nuit à 28°C. Les bactéries sont ensuite remises en suspension dans 5 ml de milieu Luria et des parties aliquotes sont étalées sur des boites de Pétri contenant un milieu minimum gélosé (décrit dans "Plant molecular biology manual" GELVIN et al., Kluwer Academic Press, 1988) en présence de 100 mg/l de rifampicine, 25 mg/l de kanamycine. Dans ces conditions, seules poussent les colonies d'Agrobacterium tumefaciens ayant intégré le plasmide pBR1. Celles-ci contiennent le gène chimérique dans un contexte permettant sa réplication.

La résistance aux deux antibiotiques des colonies sélectionnées est vérifiée en repiquant celles-ci sur le même milieu de sélection deux fois de suite. La présence du gène chimérique de l'endochitinase dans Agrobacterium tumefaciens est vérifiée par la méthode de Southern sur une préparation d'ADN total. (Lyse des cellules, purification de l'ADN par extraction à l'aide du mélange phénol/chloroforme, selon le protocole décrit par GELVIN dans l'ouvrage cité ci-dessus, coupure de l'ADN purifié à l'aide d'enzymes de restriction, électrophorèse sur gel d'agarose, transfert sur membrane et hybridation selon les techniques bien connues de l'homme de l'art).

### b) Transfert dans Agrobacterium rhizogenes

Ce transfert est réalisé de la même façon que le transfert dans Agrobacterium tumefaciens décrit en a), avec la souche Agrobacterium rhizogenes A4 décrite par GUERCHE et al. (1987) Mol. gen. genet. 206, 382.

### EXEMPLE 3 : Obtention de plantes de tabac transformées.

Le tabac Nicotiana tabacum cultivé in vitro a été infecté par Agrobacterium tumefaciens contenant le plasmide pBR1 selon la procédure de Horsch et al., bien connue de l'homme du métier (HORSH R.B et al. 1985, Science 227, 1229-1231), dont les principales étapes sont exposées ci-après.

Des disques de feuilles de plantes axéniques de tabac N. tabacum (variété Wisconsin Havana 38 sensible aux champignons pathogènes) sont incubés dans une culture d'A. tumefaciens hébergeant le plasmide pBR1. Les disques égouttés sur papier Whatman ont été transférés sur des milieux de culture en boites de Pétri afin de multiplier les cellules transformées de façon à obtenir des cals, puis produire des pousses en présence de céfotaxime (500 µg/ml) et de kanamycine (100 µg/ml). Les pousses résistantes à la kanamycine ont été ensuite transférées sur un milieu permettant l'induction des racines en présence de céfotaxime et de kanamycine. Les plantules sont ensuite repiquées en terrines dans un substrat composé de tourbe et de terreau et mises à croître en serre. Toutes les plantes transformées (génération Ro) ayant survécu aux étapes de régénération et d'acclimatation en serre se sont révélées morphologiquement normales et fertiles. Elles ont été autofécondées et ont donné des graines (génération R₁).

### EXEMPLE 4 : Analyse de l'ADN génomique des plantes de tabac transformées (génération Ro) selon la technique de SOUTHERN Blot.

L'ADN génomique de haut poids moléculaire a été isolé à partir de feuilles matures de plantes transgéniques de la génération Ro selon la méthode d'extraction à l'aide de bromure de cétyltriméthylammonium et de purification par précipitation, décrite dans l'ouvrage "Plant Molecular Biology Manual" déjà cité.

10 µg de cet ADN génomique ont été digérés toute la nuit à 37°C avec 20 unités des enzymes de restriction HindIII et EcoRI. Les fragments de restriction obtenus ont été séparés par électrophorèse sur gel d'agarose (1 %). L'ADN a été transféré selon la méthode de SOUTHERN Blot sur un filtre de nitrocellulose et hybridé avec une sonde nucléotidique comprenant la séquence du gène chimérique recombinant marquée à l'α32-dCTP par marquage au hasard (random priming). Le lavage à haute stringence a été réalisé en présence de 0,2 x SSC, 0,1 % SDS à 68°C avant l'autoradiographie. L'analyse de l'autoradiogramme permet de tirer les conclusions suivantes :
- certaines plantes ne possèdent pas de copies du gène transféré (absence de signal).
- La plupart des plantes testées contiennent au moins une copie sans réarrangement de la construction : promoteur 35S CaMV - gène chimérique de l'endochitinase - terminateur NOS.
- Certains profils suggèrent qu'il existe des réarrangements internes dans la construction, mais ces évènements sont rares.

### EXEMPLE 5 : Détermination pour les plantes de tabac transformées (générations Ro, R₁ et R₂) de l'expression de l'endochitinase recombinante.

### 1) Analyse de l'ARN messager selon la technique de Northern Blot.

L'ARN total des plantes transformées de la génération Ro a été isolé selon le protocole de VERWOERD et al., NAR, 17, 2362, 1989. Des portions de feuilles sont prélevées et broyées, puis traitées par un mélange de phénol/Tris-HCl pH 8,0/LiCl 0,1M.

Les ARN sont purifiés par traitement au chloroforme, puis au LiCl 2M et précipités.

15 µg d'ARN de chaque plante sont séparés par électrophorèse sur gel d'agarose (1,2 %) en conditions dénaturantes (Maniatis op. cit.) puis transférés sur une membrane de nitrocellulose (Hybond C-extra Amersham). Les ARN messagers (ARNm) correspondant au gène introduit sont repérés à l'aide d'une sonde oligonuléotidique de séquence **(SEQ ID NO : 9) :** 5'    AGGGCCGCCACCTGGACACTGA    3' préalablement marquée au moyen d'α32-dCTP et de la terminal transférase (Boehringer Mannheim) selon le protocole décrit dans Maniatis et al. (op. cit.).

Cette analyse a permis de mettre en évidence pour les plantes transformées la présence d'un signal d'hybridation correspondant à un ARN messager d'environ 1500 nucléotides, absent des plantes non transformées.

### 2) Détermination de l'expression de l'endochitinase.

La méthode utilisée fait appel à la visualisation par des techniques immunologiques de l'endochitinase recombinante.

a) Préparation des anticorps : une endochitinase de tomate a été purifiée jusqu'à homogénéité à partir de cals de tomate comme décrit ci-après : des cals de tomate ont été cultivés in vitro sur un milieu de Murashige et Skoog (Murashige T. et Skoog F., 1962, Physiol. Plant., 15, 473-497) contenant 0,1 mg/l d'ANA (acide naphtalène acétique) et 1 mg/l de BAP (benzylamino purine).

Des extraits cellulaires sont obtenus par broyage du matériel végétal dans une solution tampon Tris-HCL 50 mM pH 8,4 contenant 15 mM de β mercaptoéthanol et 5 % de polyvinylpolypyrrolidone.

La protéine est purifiée à partir de cet extrait par précipitation au sulfate d'ammonium, chromatographie en phase liquide selon la technique FPLC de PHARMACIA sur la colonne échangeuse de cations à base de polymère synthétique (Mono S de Pharmacia), et chromatographie d'exclusion (tamisage moléculaire) sur un agarose réticulé selon le protocole décrit ci-après :

### Protocole de purification de l'endochitinase de tomate :

ETAPE 1 : L'extrait protéique est précipité par le sulfate d'ammonium (60 % de saturation). Les protéines ayant précipité sont récupérées par centrifugation (15 000 g pendant 30 min), solubilisées dans une solution tampon (acétate d'ammonium 100 mM pH 5,2) et dialysées une nuit à 4°C contre une solution tampon acétate d'ammonium 100 mM pH 5,2.

Extemporanément, la concentration de la solution tampon de l'extrait protéique est ramenée à 10 mM par passage sur des mini-colonnes prêtes à l'emploi (PD10. Pharmacia).

ETAPE 2 : L'extrait protéique est ensuite purifié par chromatographie d'échange d'ions à base de polymère synthétique (colonne Mono-S de Pharmacia) à l'aide d'une technique FPLC (Pharmacia).

L'extrait est déposé sur la colonne Mono-S équilibrée avec un tampon acétate d'ammonium 10 mM pH 5,2. Les protéines retenues sur la colonne sont éluées par un gradient linéaire de 10 à 500 mM d'acétate d'ammonium.

ETAPE 3 : Les fractions contenant l'endochitinase de tomate sont concentrées par ultrafiltration sur membrane Centricon 10 (Amicon). La purification de la protéine est poursuivie par chromatographie (tamisage moléculaire) sur un agarose réticulé (colonne SUPEROSE 12 Pharmacia), l'élution est réalisée par une solution tampon acétate d'ammonium 500 mM pH 5,2.

A chaque étape, l'endochitinase de tomate est identifiée par son poids moléculaire (Electrophorèse sur gel de polyacrylamide en présence de SDS - révélation à l'argent) et son activité endochitinase, mesurée par une méthode radiochimique (Cf. exemple 9 ci-après) utilisant la chitine marquée comme substrat (MOLANO et al. (1977) Anal. Biochem 83, 648-656).

On a ensuite injecté à des lapins 25 µg d'endochitinase de tomate dans 500 µl d'adjuvant complet de Freund. Trois injections de rappel de 25 µg dans l'adjuvant incomplet de Freund (500 µl) ont été réalisées à 3 semaines d'intervalle. L'immunsérum a été prélevé après la dernière injection.

b) Préparation des extraits bruts de protéines de olantes de tabac transformées (génération Ro) :

Les extraits bruts de protéines ont été préparés à partir de différents tissus de la plante (racine, tige, feuille, etc...). Les fragments de tissus ont été congelés dans l'azote liquide, réduits en poudre et stockés à -20°C. La poudre a été extraite à 4°C en présence d'un tampon acétate d'ammonium 0,1 M pH 5,2 et soumise à une centrifugation à 10 000 g. La concentration des protéines totales a été déterminée sur les surnageants, appelés ci-après Les extraits bruts de protéines en suivant la technique de Bradford (Bradford, M.M., (1976) Anal. Biochem., 72, 248-254).

c) Mise en évidence par immuno-empreinte (Western Blot)

On soumet les extraits bruts de protéines de différentes plantes transformées et des plantes non transformées (témoins) à un Western Blot, technique bien connue de l'homme de l'art et décrite notamment par H. TOWBIN et al. : Proc. Ntl. Acad. Sci. USA, 76, 1972, 4350-4354, qui comprend les étapes suivantes :
- dénaturation par ébullition pendant 10 min dans un tampon dénommé tampon de charge constitué de Tris HCl 0,125 M pH 6,8, SDS 4 %, bleu de bromophénol 0,002 %, glycérol 20 %, β-mercaptoéthanol 10 % (selon le protocole décrit par LAEMMLI (U.K LAEMMLI, Nature, 227 (1970), 680-685) ;
- séparation électrophorétique des différentes protéines contenues dans le solubilisat selon le protocole décrit par LAEMMLI (U.K. LAEMMLI, Nature, 227 (1970), 680-685) ;
- électrotransfert desdites protéines contenues dans le gel sur une membrane en PVDF (selon la technique de H. T0WBIN et al. Proc. Natl. Acad. Sci. USA 76 (1979) 4350-4354).

L'immunodétection est réalisée selon un protocole qui comprend les étapes suivantes :
- saturation de la membrane PVDF sur laquelle les protéines ont été transférées par incubation au minimum 2 heures à 37°C dans une solution de gélatine à 3 %
- 3 lavages dans du tampon phosohate salin contenant 0,05 % du détergent Tween 20
- incubation (1 heure à 37°C) en présence de l'immunsérum préparé précédemment (contenant les anticorps polyclonaux reconnaissant la protéine recombinante) dilué au 1/10 000 dans ou tarpon phosphate salin
- 3 lavages dans du tampon phosphate salin contenant 0,05 % du détergent Tween 20.

Le complexe antigène-anticorps est ensuite révélé à l'aide d'un système streptavidine-biotine conjugué à la phosphatase alcaline avec le kit RPN 23 d'AMERSHAM (Blotting-détection kit) utilisé selon les indications du fabricant.

L'empreinte obtenue montre la présence d'une protéine d'environ 26 kDa pour les plantes transformées, absente des plantes témoins. (La protéine déduite de la séquence du gène chimérique, clivée de son peptide signal supposé, a une masse moléculaire d'environ 32 kDa).

L'analyse selon La technique de Northern Blot et selon celle de Western Blot a été effectuée sur 30 plantes transformées (répondant positivement au Southern Blot). 28 plantes ont montré une expression de l'ARN messager du gène chimérique en Northern Blot et une expression de l'endochitinase recombinante en Western Blot. La non-expression pour 2 plantes résulte probablement de l'insertion du gêne chimérique dans un contexte non transcrit.

L'analyse, selon la technique de Northern Blot et selon celle de Western Blot, a été également effectuée sur les plantes de la génération R₁ issues de plantes transformées de la génération Ro exprimant la protéine recombinante et sur des plantes de la génération R₂ issues de plantes de la génération R₁ exprimant la protéine recombinante. En accord avec la ségrégation Mendelienne (Cf. exemple 6 ci-après), la plupart, mais non la totalité, des plantes de la génération R₁ et de la génération R₂ expriment la protéine recombinante.

Ces résultats montrent donc la stabilité de l'insertion du gène dans les plantes de tabac et de son expression au cours des générations successives.

### EXEMPLE 6 : Analyse génétique des plantes de tabac transformées (génération R₁).

Les tabacs régénérés (génération Ro) en présence de kanamycine ont été autopollinisés. Les graines matures (génération R₁) sont récoltées et stockées en tubes Eppendorf à 4°C. Les graines sont stérilisées en surface à l'aide d'une solution aqueuse d'hypochlorite de calcium à 2 %. Les graines sont ensuite rincées à l'eau stérile, séchées pendant 24 h dans une hotte à flux laminaire sur du papier filtre et mises à germer sur du milieu Murashige et Skoog gélosé, complémenté par 100 µg/ml de kanamycine (le gène de résistance à la kanamycine lié au gène chimérique complet et transféré au tabac en même temps que celui-ci sert ici de marqueur de sélection).

L'analyse génétique a été effectuée sur les descendances de 16 plantes transformées (de la génération Ro) choisies parmi les 28 plantes exprimant l'endochitinase recombinante (Cf. exemple 4), abrégées Tn (n étant le n° attribué à La plante) et d'une plante témoin Nicotiana tabacum var Wisconsin Havana 38 non transformée, abrégée WH 38. Le nombre d'individus observés (effectif total) varie selon les descendances de 27 à 139. Le taux de germination est élevé (de l'ordre de 95 %) et comparable pour toutes les plantes étudiées.

Deux types de phénotypes sont observés au moment de la germination des graines :
- des plantules résistantes à la kanamycine qui poussent bien en présence de 100 µg/ml de kanamycine et possèdent un système racinaire développé et des feuilles vertes,
- des plantules sensibles à la kanamycine, correspondant soit à des plantes qui ne développent pas de racines et qui produisent des feuilles blanches, soit à des plantes pour lesquelles le système racinaire est réduit et qui produisent des feuilles avec des zones blanches.

On définit la ségrégation génétique comme le rapport du nombre de plantes résistantes sur le nombre de plantes sensibles à la kanamycine.

Le tableau 1 ci-après rassemble les résultats obtenus :

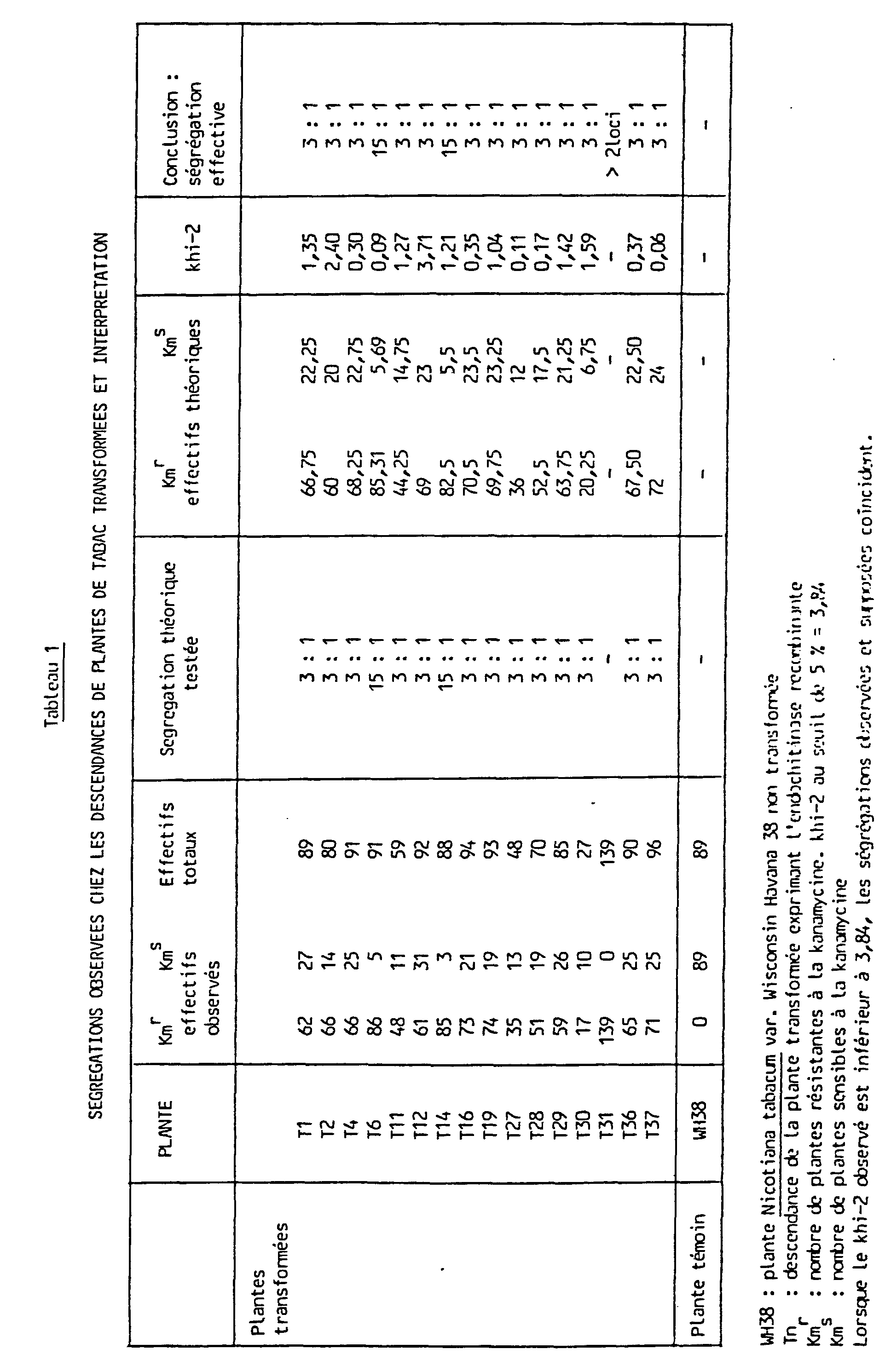

L'analyse statistique des résultats rassemblés dans le tableau 1 ci-dessus montre que le caractère : résistance à la kanamycine, génétiquement lié au caractère conféré par le gène chinérique de l'endochitinase se comporte comme un caractère Mendélien dominant simple (ségrégation Mendélienne 3/1 ou 15/1) présent en un seul locus (une copie ou plusieurs copies du gène proches : 3/1), en deux loci (deux ensembles distants génétiquement comprenant chacun une copie ou plusieurs copies du gène proches génétiquement : 15/1) ou en plus de deux loci (cas de la plante T31).

Le nombre de loci dans chaque plante a été confirné par l'analyse des descendances de la génération R₂.

### EXEMPLE 7 : Mesure de la résistance aux champignons pathogènes des plantes transformées (génération R₁)

Les plantules de la génération R₁ résistantes à la kanamycine issues des 16 plantes transformées choisies, d'une plante Nicotiana tabacum var. Wisconsin Havana 38 sensible à Chalara elegans (également appelé Thielaviopsis basicola), abrégée WH38, et d'une plante Nicotiana tabacum var. Paraguay 49, abrégée P49, génétiquement tolérante à ce champignon, ont été transférées en serre pour évaluation de leur résistance à ce champignon. Ce dernier a été choisi car il est représentatif des champignons pathogènes du tabac possédant de la chitine dans leur paroi. L'étude a porté sur des effectifs de plantules variant de 15 à 36 selon les plantes. Le protocole choisi dans cette étude est décrit ci-après :

Les plantules sont cultivées en godets (3 x 3 cm). A l'apparition de la 5ème feuille, les plantes sont inoculées en déposant au niveau du collet une suspension d'endoconidies 5 x 10⁵ spores/ml). Les endoconidies sont prélevées sur des cultures mycéliennes de ce champignon entretenues sur le milieu "potato dextrose agar" (Difco) à 22°C et à l'obscurité. On évalue la résistance à Chalara elegans en attribuant une note 45 jours après l'inoculation. Les plantes sont notées selon les symptômes d'infection et selon leur niveau de développement végétatif par rapport à un témoin non inoculé (ce témoin est une plante WH38 non inoculée pour les plantes issues des 16 plantes transformées choisies et de la plante WH38 et une plante P49 non inoculée pour les plantes issues de la plante P49). Les classes sont définies selon les critères suivants :

Note 0 : plante morte ; note 1 : bourgeon terminal encore vert, système racinaire détruit ; note 2 : développement des plantes ne dépassant pas 25 % de celui du témoin, système racinaire entièrement nécrosé ; note 3 : développement de plantes atteignant 50 % du développement du témoin, système racinaire présentant des parties saines ; note 4 : développement des plantes identiques au témoin.

L'indice de résistance de la descendance d'une plante transformée représente la moyenne des notes effectuées sur les plantules issues de cette plante.

Le tableau 2 ci-après rassemble les résultats obtenus :

**Tableau 2**

| MESURE DE LA RESISTANCE AUX CHAMPIGNONS PATHOGENES DE DESCENDANCES DE PLANTES DE TABAC TRANSFORMEES | | | |
|---|---|---|---|
| | Plante | Effectif testé | Indice de résistance de la descendance |
| Plantes transformées | | | |
| | T1 | 32 | 2,030 |
| | T2 | 31 | 0,065 |
| | T4 | 36 | 1,200 |
| | T6 | 34 | 0,743 |
| | T11 | 35 | 2,514 |
| | T12 | 33 | 1,632 |
| | T14 | 26 | 1,769 |
| | T16 | 36 | 1,750 |
| | T19 | 36 | 1,416 |
| | T27 | 20 | 0,050 |
| | T28 | 27 | 1,444 |
| | T29 | 36 | 1,750 |
| | T30 | 15 | 2,666 |
| | T31 | 36 | 2,888 |
| | T36 | 36 | 1,686 |
| Plantes | WH38 | 34 | 0,044 |
| témoins | P49 | 34 | 2,823 |
| WH38 : plante Nicotiana tabacum var. Wisconsin Havana 38 non transformée P39 : plante Nicotiana tabacum var Paraguay 49 Tn : descendance de la plante transformée exprimant l'endochitinase recombinante. | | | |

On constate à la lecture du tableau ci-dessus que toutes les descendances des plantes transformées Tn présentent un indice de résistance supérieur à celui de la descendance de la plante témoin WH38 (plante non transformée) et parfois proche de, voire supérieur à, celui de la descendance de la plante témoin P49 génétiquement résistante.

### EXEMPLE 8 : Obtention de plantes de colza transformées

La transformation est réalisée selon le protocole de P. GUERCHE et al. (P. GUERCHE et al. 1987, Mol. Gen. Genet., 206, 382). Les différents milieux de culture sont ceux décrits par Pelletier et al. (Pelletier et al., 1983, Mol. gen. genet., 191, 244). Leur composition sera explicitée par la suite (tableau 3).

### a) Obtention de racines transformées

Des segments de tige sont prélevés sur l'extrémité apicale de plantes de colza (Brassica napus : variétés de printemps Brutor et Westar et variété d'hiver) de 1 m de haut environ. Ces segments sont stérilisés en surface, rincés dans de l'eau stérile, découpés en segments de 1,5 cm de Long environ et placés dans un tube contenant le milieu A.

L'inoculation de l'extrémité de ce segment est effectuée par dépôt d'une suspension de la souche d'Agrobacterium rhizogenes contenant le plasmide pBR1.

Des racines transformées apparaissent sur le segment de tige au bout de 1 à 2 semaines, elles sont prélevées et placées sur le milieu B gélosé (15 g/l) et complémentées par 500 µg de céfotaxime/ml.

### b) Obtention des cals transformés

Des fragments de racines sont incubés pendant 15 jours sur le milieu D contenant 3 mg/l d'acide 2,4-dichlorophénoxyacétique, puis transférés sur le même milieu gélosé (15 g/l) en vue de la multiplication des cellules transformées de façon à obtenir des cals et à fournir des extraits bruts destinés à la purification de la protéine recombinante (Cf. exemple 10 ci-après).

### c) Régénération de plantes transformées

Des fragments de racines sont incubés pendant 15 jours sur le milieu D contenant 3 mg/l d'acide 2,4-dichlorophénoxyacétique, puis placés sur le milieu RCC d'induction de bourgeons. Des plantes racinées sont ensuite obtenues par passage des bourgeons sur les milieux F et G.

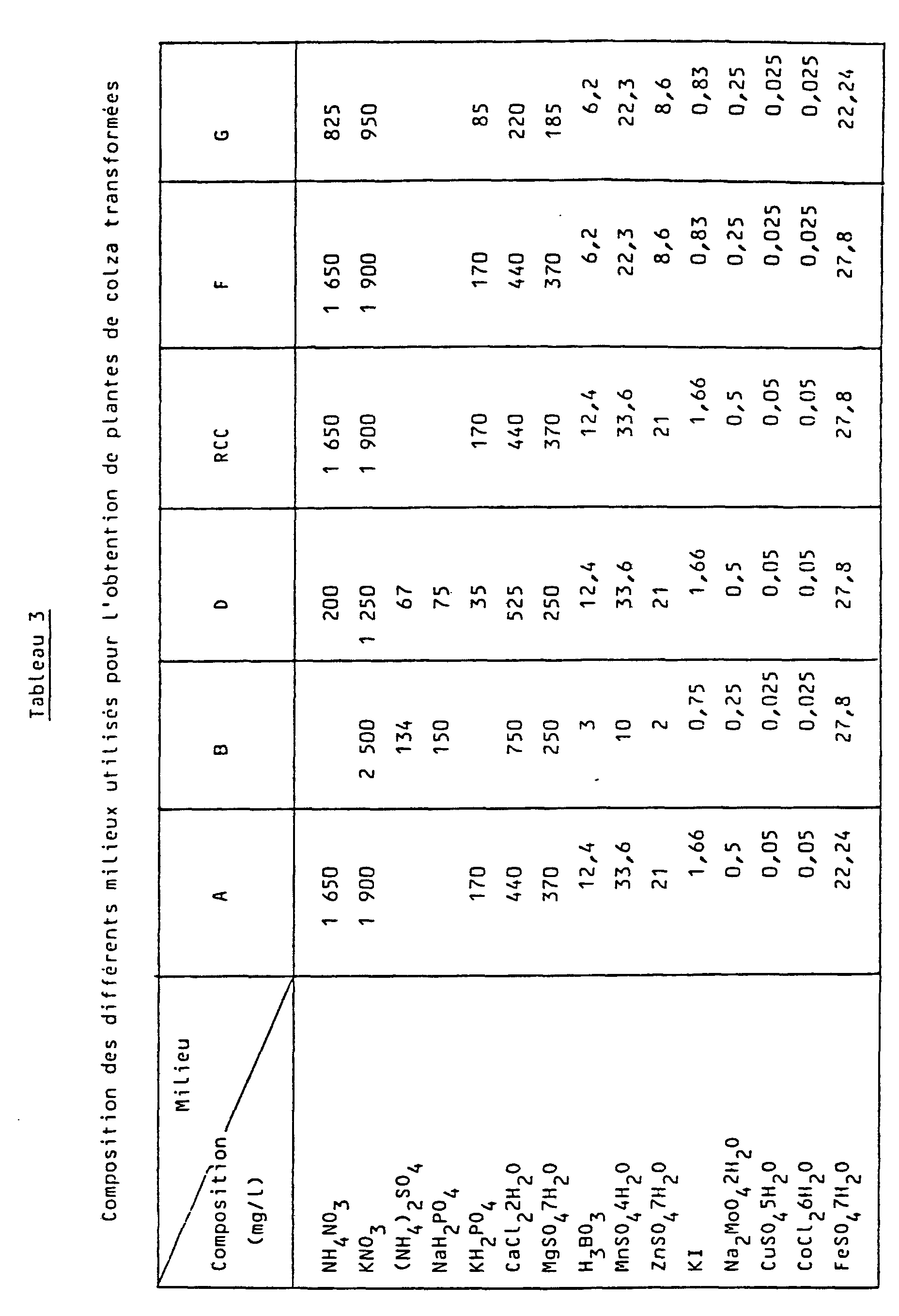

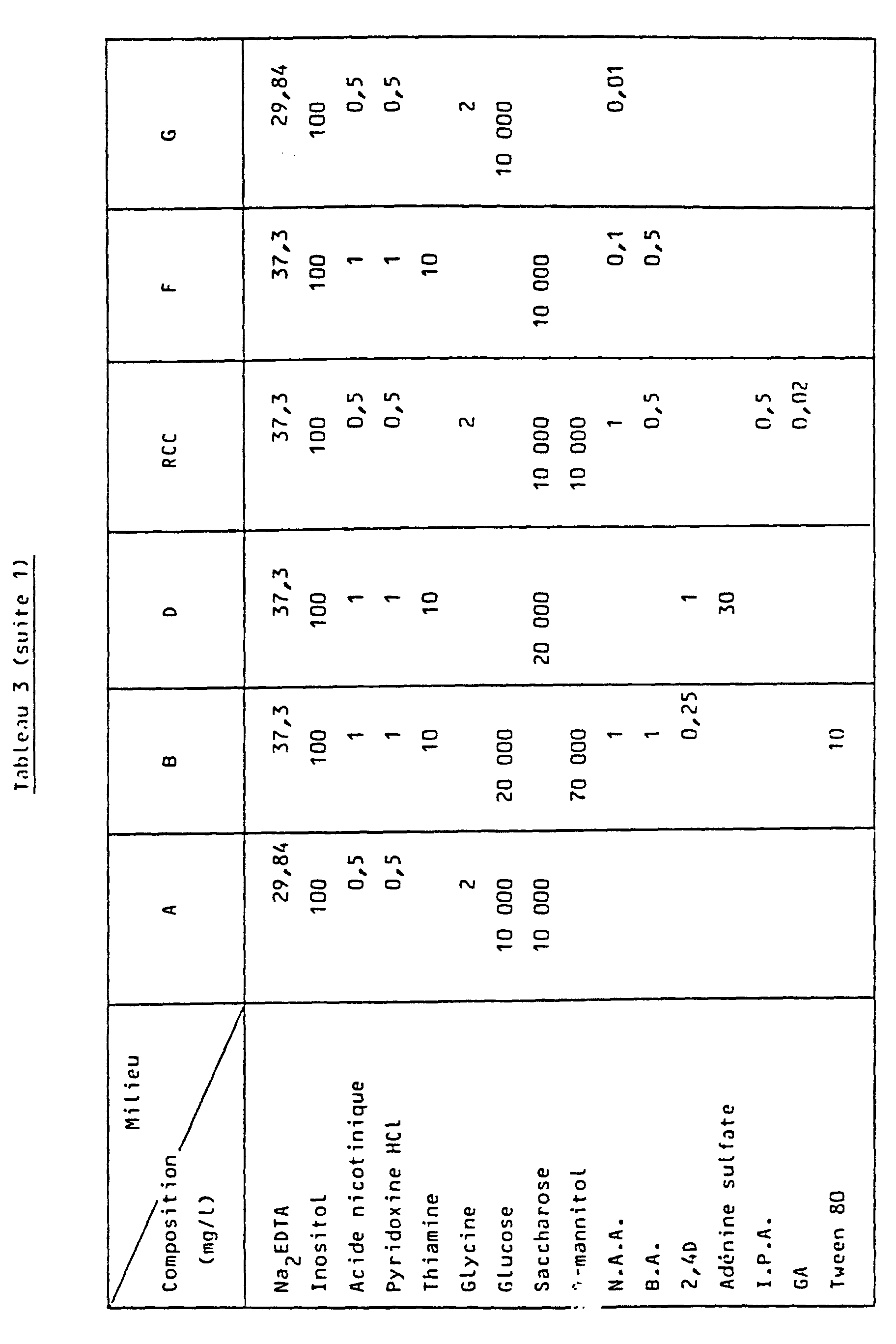

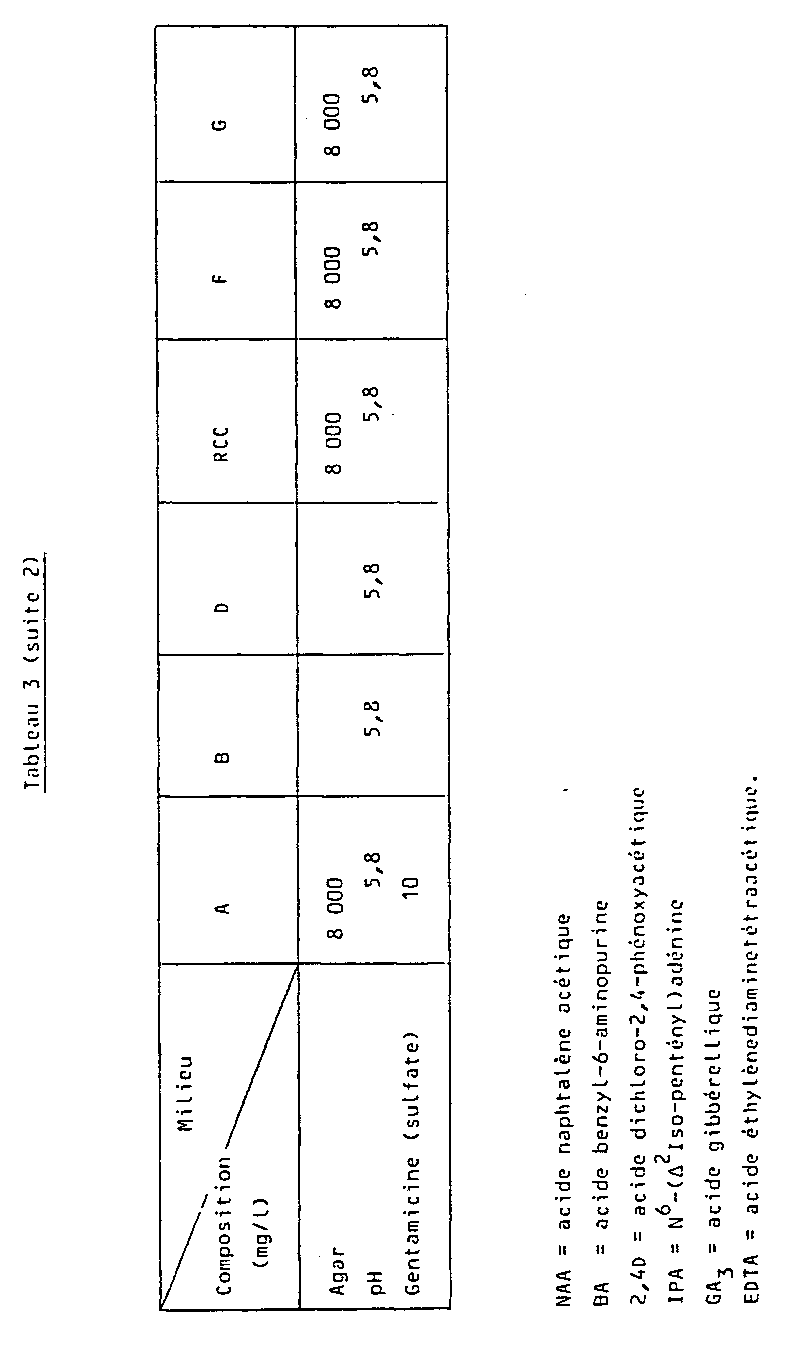

### EXEMPLE 9 : Analyse de L'ADN génomique des plantes de colza transformées (génération Ro) et détermination pour ces dernières et de leurs descendances de l'expression de l'endochitinase recombinante.

### 1) Analyse de l'ADN génomique selon la technique de Southern Blot

L'analyse de l'ADN génomique selon la technique de Southern Blot, effectuée dans les conditions décrites dans l'exemple 4, a permis de constater que la plupart des plantes testées contiennent au moins une copie sans réarrangement de la construction promoteur 35S CaMV - gène chimérique de l'endochitinase - terminateur NOS.

### 2) Analyse de l'ARN messager selon la technique de Northern Blct

L'analyse de l'ARN messager selon la technique de Northern Blot, effectuée dans les conditions décrites dans l'exemple 5, n'a été mise en oeuvre que pour quelques plantes, l'analyse selon la technique de Western Blot étant plus rapide pour fournir l'information attendue. Elle a permis de mettre en évidence pour les plantes transformées analysées la présence d'un ARN messager d'environ 1 500 nucléotides, absent des plantes non transformées.

### 3) Détermination de l'expression de l'endochitinase recombinante par Western Blot

L'analyse en Western Blot, effectuée dans les conditions et à l'aide des anticorps décrits dans l'exemple 5 sur les extraits bruts de protéines de plantes de colza transformées (préparés comme décrit dans l'exemple 5 pour les extraits bruts de protéines de plantes de tabac transformées) a permis de visualiser la protéine recombinante.

L'empreinte obtenue montre la présence d'une protéine d'environ 26 kDa pour les plantes transformées, absente des plantes témoins (la protéine déduite de la séquence du gène chimérique, clivée de son peptide signal a une masse moléculaire d'environ 32 kDa) et également la présence d'une protéine d'environ 38 kDa reconnue par les anticorps, présente également dans les plantes non transformées. Cette dernière est une endochitinase endogène (Atta K.K. et al., 1988, Abstracts du Second International Congress of Plant Molecular Biology, Jerusalem) qui présente des caractéristiques sérologiques communes avec celles de l'endochitinase recombinante.

L'analyse selon la technique de Western Blot a été effectuée sur 42 plantes transformées (répondant positivement au Southern Blot). 38 plantes ont montré une expression de l'endochitinase recombinante. La non-expression observée pour 4 plantes résulte probablement de l'insertion du gène chimérique dans un contexte non transcrit.

L'analyse selon la technique de Western Blot a égalenent été effectuée sur des plantes de la génération R₁ issues de plantes transformées de la génération Ro exprimant l'endochitinase recombinante. En accord avec les lois de la génétique pour l'hérédité des diploides, la plupart mais non la totalité de celles-ci expriment la protéine recombinante.

Ces résultats montrent la stabilité de l'insertion du gène dans les plantes de colza et de son expression au cours des générations.

### EXEMPLE 10 : Purification de l'endochitinase recombinante des cals de colza transformés (génération Ro), mesure de son activité enzymatique et détermination de sa séquence aminoterminale.

### 1) Purification de l'endochitinase recombinante :

La protéine recombinante a été purifiée à partir des extraits bruts de protéines de cals de colza transformés, par précipitation au sulfate d'ammonium, chromatographie en phase liquide FPLC sur une colonne échangeuse de cations à base de polymère synthétique et chromatographie d'exclusion (tamisage moléculaire) sur un agarose réticulé, selon le protocole décrit ci-après :

### Protocole de purification de l'endochitinase recombinante

ETAPE 1 : L'extrait protéique est précipité par le sulfate d'ammonium (60 % de saturation). Les protéines ayant précipité sont récupérées par centrifugation (15 000 g pendant 30 min), solubilisées dans une solution tampon (acétate d'ammonium 100 mM pH 5,2) et dialysées une nuit à 4°C contre une solution tampon acétate d'ammonium 100 mM pH 5,2.

Extemporanément, la concentration de la solution tampon de l'extrait protéique est ramenée à 10 mM par passage sur des mini-colonnes prêtes à l'emploi (PD10. Pharmacia).

ETAPE 2 : L'extrait protéique est ensuite purifié par chronatographie d'échange d'ions à base de polymère synthétique (colonne Mono-S de Pharmacia) à l'aide d'une technique FPLC (Pharmacia).

L'extrait est déposé sur la colonne Mono-S équilibrée avec un tampon acétate d'ammonium 10 mM pH 5,2. Les protéines retenues sur la colonne sont éluées par un gradient linéaire de 10 à 500 mM d'acétate d'ammonium.

ETAPE 3 : Les fractions contenant l'endochitinase recorbinante sont concentrées par ultrafiltration sur membrane Centricon 10 (Amicon). La purification de la protéine est poursuivie par chromatographie d'exclusion (tamisage moléculaire) sur un agarose réticulé (colonne SUPEROSE 12 PHARMACIA), l'élution étant réalisée par une solution tampon acétate d'ammonium 500 mM pH 5,2.

A chaque étape, l'endochitinase de tomate est identifiée par son poids moléculaire (Electrophorèse sur gel de polyacrylamide en présence de SDS-révélation à l'argent), par son immuno-empreinte (Cf. exemple 5 c)) et son activité endochitinase, mesurée par une méthode radiochimique décrite ci-après utilisant la chitine marquée comme substrat (MOLANO et al. (1977) Anal. Biochem 83, 648-656).

### 2) Mesure de l'activité enzymatique de l'endochitinase recombinante.

### a) Méthode

L'activité endochitinase est mesurée par une méthode radiochimique utilisant La chitine marquée au tritium comme substrat selon un protocole décrit par MOLANO et al., 1977, Anal. Biochem 83, 648-656, résumé ci-après.

A 50 µl de chitine tritiée (50 kBq/ml), lavés préalablement par 4 centrifugations successives et renouvellement du solvant, on ajoute 50 µl de fraction contenant l'endochitinase recombinante puis 250 µl de solution tampon acétate de sodium 0,2 M pH 4,5. Après 45 min d'incubation à 20°C, la réaction est arrêtée par addition de 100 µl d'acide trichloroacétique 20 %. Après centrifugation (10 000 g pendant 10 min), la quantité de radioactivité solubilisée dans 100 µl de surnageant est mesurée en scintillation liquide.

A chaque étape de purification selon la méthode décrite ci-dessus, la protéine recombinante (identifiée grâce à son poids moléculaire et sa réaction positive avec les anticorps polyclonaux contre l'endochitinase de tomate) montre une activité endochitinase.

### b) Résultats

Les activités spécifiques mesurées à l'issue de l'étape 1, de l'étape 2 et de l'étape 3 sont respectivement de 135, 7 416 et 32 193 cpm/µg de protéine.

### 3) Détermination de la séquence aminoterminale de l'endochitinase recombinante mature.

Après purification de l'endochitinase recombinante selon le protocole décrit ci-dessus, le séquençage de L'extrémité aminoterminale a été réalisé. Les échantillons à traiter sont portés à La surface d'un filtre PVDF (polyvinylidenedifluoride) par électrotransfert selon la méthode décrite par H. TOWBIN et al. Proc. Ntl. Acad. Sci. USA (1979), 4350-4354, après électrophorèse sur gel de polyacrylamide en présence de SDS. Le filtre est introduit dans un séquenceur de protéines (modèle 470 A commercialisé par la Société Applied Biosystems (E.U.A.)) équipé d'un chromatographe (modèle 430 d'Applied Biosystems) qui analyse en continu les dérivés phénylthiohydantoïques formés, après chaque cycle de dégradation.

La séquence aminoterminale déterminée **(SEQ ID NO : 10)** est représentée ci-après, le symbole Xaa représentant un acide aminé non déterminé :

Gly-Gly-Xaa-Leu-Gly-Ser-Val-Ile-Ser-Asn-Xaa-Met-Phe-Xaa-Gln-Met-
Leu-Lys-Xaa-Arg

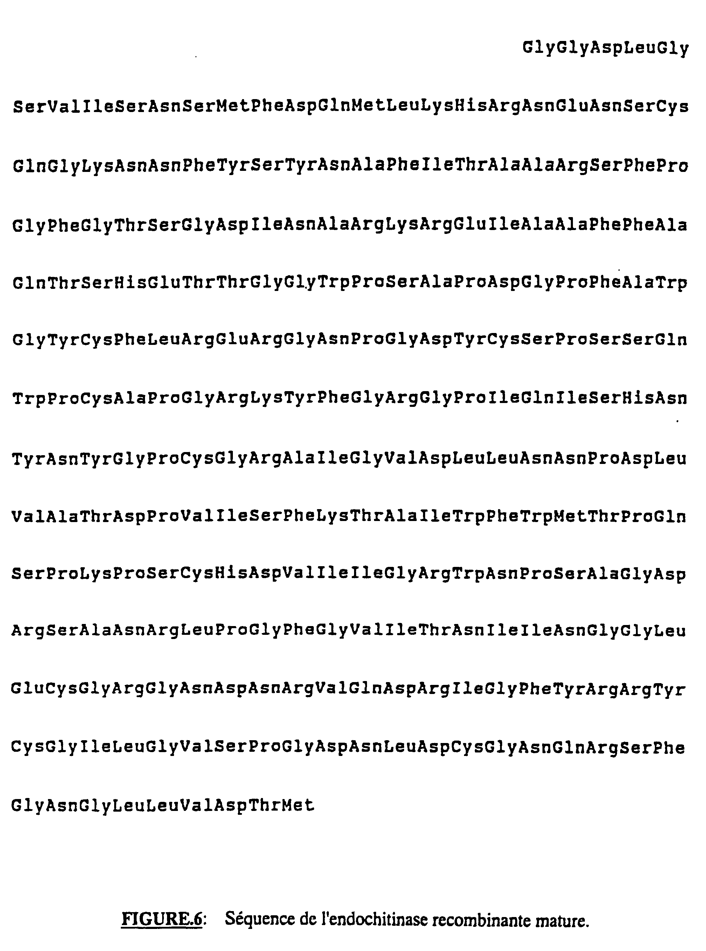

On constate que le début de la séquence de la protéine mature, **SEQ ID NO : 1** représentée sur la figure 6, correspond au 76ème acide aminé à partir de la méthionine aminoterminale déduite de la séquence du gène chimérique **(SEQ ID NO : 11),** telle que représentée sur la figure 4.

La protéine traduite à partir de l'ARN messager codé par le gène chimérique subit un clivage de son peptide signal, supposé de 24 acides aminés (G. von Heijne, 1986, Nucl. Ac. Res., 14, 483-490), puis une maturation provoquant le clivage d'un peptide amino-terminal de 51 acides aminés.

La séquence du gène chimérique contient donc les informations nécessaires à la synthèse d'une protéine de type preproenzyme, qui est maturée en endochitinase active.

### EXEMPLE 11 : Analyse génétique des plantes de colza transformées (génération R₁)

Les colzas régénérés (génération R₀) ont été autopolinisés. Les graines matures (génération R₁) sont récoltées et stockées dans des sachets. Ces graines sont ensuite semées dans des bacs sur de la vermiculite, puis les jeunes plantes sont répiquées individuellement en pot de 2 l contenant du terreau horticole. L'expression de la protéine recombinante est mise en évidence par la technique de Western blot sur des jeunes feuilles (cf. paragraphe 3 de l'exemple 9, après extraction des protéines selon le protocole décrit dans l'exemple 5b).

En accord avec les lois de la génétique pour l'hérédité des diploïdes, la plupart, mais non la totalité de ces plantes expriment la protéine recombinante.

15 des descendances de colzas transformés ont été analysées statistiquement selon le protocole décrit dans l'exemple 7. Les résultats obtenus montrent que le caractère expression du gène chimérique de l'endochitinase se comporte comme un caractère Mendélien dominant simple présent en un seul locus (12 descendances sur 15 présentent la ségrégation Mendélienne 3/1) ou en deux loci (3 descendances sur 15 présentent la ségrégation Mendélienne 15/1).

### EXEMPLE 12 : Mesure de la résistance aux champignons pathogènes des plantes de colza transformées (génération R₂)

Les plantes de génération R₁, exprimant la proteine recombinante sont autopollinisées. Les graines obtenues de génération R₂, sont mises à germer comme décrit dans l'exemple 11.

La résistance des plantes de colza exprimant la protéine recombinante est estimée sur Les plantes-de génération R₂ par inoculation en chambre de culture à l'aide d'Alternaria brassicae, champignon représentatif des champignons pathogènes du colza, selon le protocole décrit par Bains et Tewari, 1987, Physiol. Mol. Plant. Pathol. 30:259 et résumé ci-après.

Des jeunes plantes de colza âgées de 21 jours sont inoculées à l'aide d'une suspension de spores déposée sur la nervure centrale de la première feuille, préalablement blessée à l'aide d'une aiguille. Deux semaines plus tard, l'extension de la nécrose résultant du développement du champignon parasite est mesurée.

Les résultats obtenus à partir des descendances de 10 plantes transformées, montrent que trois descendances présentent une résistance notablement accrue, proche de celle d'une variété de moutarde Sinapis alba, (bains et tewari, référence ci-dessus), génétiquement résistante à Alternaria brassicae.

### EXEMPLE 13 : Obtention des racines de tournesol transformées.

Des segments de pétioles sont prélevés sur des plantes de tournesol Helianthus annuus (variété Euroflor Rustica Semences) âgées de 6 à 10 semaines. Les segments sont désinfectés par trempage pendant 30 min dans une solution d'hypochlorite de calcium à 1 %.

Les segments de pétioles sont ensuite placés dans un tube contenant du milieu de culture de Murashige et Skoog gélosé. L'inoculation de l'extrémité de ces segments est effectuée par dépôt d'une suspension de la souche d'Agrobacterium rhizogenes contenant le plasmide pBR1.

Des racines transformées apparaissent ensuite sur le segment de pétiole au bout de 1 mois environ. Ces racines sont prélevées et placées sur le milieu M gélosé (milieu M auquel on a ajouté 6 g/l d'agarose), complémenté par 500 Dg de céfotaxime/ml. La composition du milieu M est explicitée par la suite (tableau 4). Ces racines sont repiquées chaque semaine pendant 4 semaines sur le même milieu. Elles sont ensuite transférées sur le milieu M liquide, afin d'obtenir la production de racines en quantité suffisante pour analyser l'expression de la protéine recombinante par la technique de Western blot selon le protocole décrit dans le paragraphe 3 de l'exemple 9. Les extraits bruts de protéines destinés à cette analyse sont préparés selon la technique décrite dans l'exemple 5. Les empreintes obtenues montrent la présence d'une protéine du poids moléculaire attendu (26 kDa) pour les racines transformées, absente dans les racines et feuilles de plantes de tournesol témoins (plantes non transformées).

**Tableau 4**

| COMPOSITION DU MILIEU DE CULTURE M UTILISE POUR LA CULTURE DES RACINES TRANSFORMEES DE TOURNESOL | |
|---|---|
| | Composition mg/ml |
| NH₄NO₃ | 330 |
| KNO₃ | 380 |
| KH₂PO₄ | 170 |
| MgSO₄ | 370 |
| CaCl₂ | 440 |
| H₃BO₃ | 6,3 |
| MnSO₄, 4H₂O | 22,3 |
| ZnSO₄, 7H₂O | 1,6 |
| KI | 0,83 |
| Na₂MoO₄, 2H₂O | 0,25 |
| CuSO₄, 5H₂O | 0,025 |
| CoCl₂, 6H₂O | 0,025 |
| Pyridoxine HCl | 0,1 |
| Acide nicotinique | 0,1 |
| Glycine | 0,4 |
| Inositol | 20 |
| Thiamine | 0,02 |
| Saccharose | 30,000 |
| Citrate de fer | 200 |

### LISTE DES SEQUENCES

**SEQ ID NO : 1**
   TYPE DE SEQUENCE : acides aminés
   LONGUEUR DE LA SEQUENCE : 254 acides aminés
   PROPRIETES : protéine à activité endochitinase 
Gly Gly Asp Leu Gly Ser Val Ile Ser Asn Ser Met Phe Asp Gln Met
1               5                   10                  15

Leu Lys His Arg Asn Glu Asn Ser Cys Gln Gly Lys Asn Asn Phe Tyr
            20                  25                  30

Ser Tyr Asn Ala Phe Ile Thr Ala Ala Arg Ser Phe Pro Gly Phe Gly
        35                  40                  45

Thr Ser Gly Asp Ile Asn Ala Arg Lys Arg Glu Ile Ala Ala Phe Phe
    50                  55                  60

Ala Gln Thr Ser His Glu Thr Thr Gly Gly Trp Pro Ser Ala Pro Asp
65                  70                  75                  80

Gly Pro Phe Ala Trp Gly Tyr Cys Phe Leu Arg Glu Arg Gly Asn Pro
                85                  90                  95

Gly Asp Tyr Cys Ser Pro Ser Ser Gln Trp Pro Cys Ala Pro Gly Arg
            100                 105                 110

Lys Tyr Phe Gly Arg Gly Pro Ile Gln Ile Ser His Asn Tyr Asn Tyr
        115                 120                 125

Gly Pro Cys Gly Arg Ala Ile Gly Val Asp Leu Leu Asn Asn Pro Asp
    130                 135                 140

Leu Val Ala Thr Asp Pro Val Ile Ser Phe Lys Thr Ala Ile Trp Phe
145                 150                 155                 160

Trp Met Thr Pro Gln Ser Pro Lys Pro Ser Cys His Asp Val Ile Ile
                165                 170                 175

Gly Arg Trp Asn Pro Ser Ala Gly Asp Arg Ser Ala Asn Arg Leu Pro
            180                 185                 190

Gly Phe Gly Val Ile Thr Asn Ile Ile Asn Gly Gly Leu Glu Cys Gly
        195                 200                 205

Arg Gly Asn Asp Asn Arg Val Gln Asp Arg Ile Gly Phe Tyr Arg Arg
    210                 215                 220

Tyr Cys Gly Ile Leu Gly Val Ser Pro Gly Asp Asn Leu Asp Cys Gly
225                 230                 235                 240

Asn Gln Arg Ser Phe Gly Asn Gly Leu Leu Val Asp Thr Met
                245                 250
**SEQ ID NO : 2**
   TYPE DE SEQUENCE : acides aminés
   LONGUEUR DE LA SEQUENCE : 51 acides aminés
   PROPRIETES : séquence située immédiatement en amont de la séquence **SEQ ID NO : 1** 
Gln Asn Cys Gly Ser Gln Gly Gly Gly Lys Val Cys Ala Ser Gly Gln Cys
    -50                 -45                     -40                     -35

Cys Ser Lys Phe Gly Trp Cys Gly Asn Thr Asn Asp His Cys Gly Ser Gly
                -30                     -25                     -20

Asn Cys Gln Ser Gln Cys Pro Gly Gly Gly Pro Gly Pro Gly Pro Val Thr
        -15                     -10                     -5          -1
**SEQ ID NO : 3**
   TYPE DE SEQUENCE : acides aminés
   LONGUEUR DE LA SEQUENCE : 24 acides aminés
   PROPRIETES : séquence située immédiatement en amont de la séquence **SEQ ID NO : 2** 
Met Arg Arg Thr Ser Lys Leu Thr Thr Phe Ser Leu Leu Phe Ser Leu Val
-75                 -70                 -65                 -60

Leu Leu Ser Ala Ala Leu Ala
            -55         -52
**SEQ ID NO : 4**
   TYPE DE SEQUENCE : nucléotidique
   LONGUEUR DE LA SEQUENCE : 1153 paires de bases
   NOMBRE DE BRINS : à double brin
   TYPE DE MOLECULE : ADN génomique
   PROPRIETES : partie codante du gène chimérique, codant pour une protéine à activité endochitinase qui comprend la séquence **SEQ ID NO : 1**
   CARACTERISTIQUES : de 443 à 521 paires de bases : intron 1 de 676 à 756 paires de bases : intron 2 
ATGAGGCGAA CTTCTAAATT GACTACTTTT TCTTTGCTGT TTTCTCTGGT TTTGCTGAGT 60
GCTGCCTTGG CACAGAATTG TGGTTCACAG GGCGGAGGCA AAGTTTGTGC GTCGGGACAA 120
TGTTGCAGCA AATTCGGGTG GTGCGGTAAC ACTAATGACC ATTGTGGTTC TGGCAATTGT 180
CAAAGTCAGT GTCCAGGTGG CGGCCCTGGT CCTGGTCCTG TTACTGGTGG GGACCTCGGA 240
AGCGTCATCT CAAATTCTAT GTTTGATCAA ATGCTTAAGC ATCGTAACGA AAATTCTTGT 300
CAAGGAAAGA ATAATTTCTA CAGTTACAAT GCCTTTATTA CTGCTGCTAG GTCTTTTCCT 360
GGCTTTGGTA CAAGTGGTGA TATCAATGCC CGTAAAAGGG AAATTGCTGC TTTCTTTGCC 420
CAAACCTCCC ATGAAACTAC TGGTATGTGT ATAACCATTC ACATCGAACC ATTAAAATAT 480
AATTTCATTT TATTTTATTT AGTAATTGAT TATATATGTA GGAGGATGGC CTTCCGCACC 540
TGATGGACCA TTCGCATGGG GTTACTGTTT CCTTAGAGAA CGAGGTAACC CCGGTGACTA 600
CTGTTCACCA AGTAGTCAAT GGCCTTGTGC ACCTGGAAGG AAATATTTCG GACGAGGCCC 660
AATCCAAATT TCACAGTAAG CTACATAAAT CTATATATGG TAAAATTTGA TGAACTTGTA 720
GTGTCTAATT ACGTGTATTT TGACATTTCA AAACAGCAAC TACAACTATG GGCCATGTGG 780
AAGAGCCATC GGAGTGGACC TTTTAAACAA TCCTGATTTA GTAGCCACAG ACCCAGTCAT 840
CTCATTCAAG ACTGCTATCT GGTTCTGGAT CACCCCTCAA TCACCAAAGC CTTCTTGCCA 900
CGATGTCATC ATTGGAAGAT GGAACCCATC TGCCGGTGAC CGATCAGCCA ATCGTCTTCC 960
TGGATTTGGT GTCATCACAA ACATCATCAA TGGGGGCCTC GAATGTGGTC GTGGCAATGA 1020
CAATAGGGTC CAGGATCGCA TTGGGTTTTA CAGGAGGTAT TGCGGTATTC TTGGTGTTAG 1080
TCCTGGTGAC AATCTTGATT GCGGAAACCA GAGATCTTTT GGAAACGGAC TTTTAGTCGA 1140
TACTATGTAA TGA 1153
**SEQ ID NO : 5**
   TYPE DE SEQUENCE : nucléotidique
   LONGUEUR DE LA SEQUENCE : paires de bases
   NOMBRE DE BRINS : à double brin
   TYPE DE MOLECULE : ADN génomique
   PROPRIETES : partie 5' du gêne de l'endochitinase de tomate
   CARACTERISTIQUES : 1^{re} paire de bases : site EcoRI
      2006° paire de bases : site StyI
      2172°paire de bases : site DralI (1)
      2934°paire de bases : site DralI (2)
      3007° paire de bases : site HindIII
      de 1942 à 3007 paires de bases : partie 5'
      de l'endochitinase de tomate
      de 2384 à 2462 paires de bases : intron 1
      de 2617 à 2697 paires de bases : intron 2 
GAATTCATAT TTATTTTAAA AAAATATTTT CAACTTCAAA AATATATTTT TTTCACGCCT 60
ACCCTCGACC CCCCTCCCGC ACCCTTACCC GCCCTCTACC AACCCCCCCC CCCCCAAAAA 120
AAAATAAATT AAACTTTACT TTTAAAAATA TTTTCAACTT CAAAATTTCA TTTTTTTTCA 180
TCCCTACCCT CGACCACCCC CACCCTCCCG CTAAAAAATA AAAGTTTAAG TTTGTTTTTG 240
AAAAGTATTT TCAACTTCAA AAATTCATTT TTTCACCCCT AACCTCAACC CCCCACCCAC 300
ATTCCCACCC CAATTTTTTT TTTTAAGTTT GTTGTTAAAA AATATTTTCT ACTTCAAAAT 360
TTCATTTCAC CCTTTCCCCC CCCCCTCCCC AAACCCCACC CCACCCCCCC ACCCCCCAAA 420
AAAAATATTT AAATTTGTTT TTAAAAAATA TTTTCAATTT CAAAATTTTA TTTTCTATTC 480
TAGTAAAAAT AAAAGATATA TCTCAAAAAC ATTTTTTACT TATTCAAAAA CCAAACACTC 540
TTTTCCAGAA AAAATTTCTA TTCACCAACC AAATATGAGA AAATAAATCA AAATCTAGTT 600
ATTTTAGAAA ATGTTTTCCT ACATATCAAA CACACCCAAT GTCTTCATTA ATGTGTTCAG 660
ATTTATTTTA TGTCAACTTG GTCGCTATGT TATATGAATT AGCCACACAA ATTCAATTTA 720
ATTGCACATT ACCACTATTT TGTAGTTCAC GTAGAAATTA AAGTTCATCA CAACAAAATA 780
AATATTGGGC GCACGGGCGA CTCCCCACTA GTATCACTCA GAAATCACAA TAAAGTATTA 840
AATTTTGTCA AAATTCTTTA TCCGTATTAA GAAATCTTTG AAGTCTGAAT ACATATAAAT 900
TCATAATTCA TAAATTTCAA ATTTCTCTTA GTAATTTTTA TTGAGTTATT AATTTCATTT 960
AAACAAATTC ATTGTACTTT GTAAATACTC CTAATTTGTA TGATTTTGGA CTCATGTAAG 1020
GAAACCTTAT CAAATTAAGT ATGGAGTTAA AGGGGAAGAG TAGAATTAGC AGCCCAAAGA 1080
TACACTTTCA AATTATGTAA GTTTGACCCA GCCTGCCCTA TTTCTTCTAG CACCAGCTGC 1140
TACCTTATAT AATTACTTTA ATTTGAAAAT GTCATCAATA TCATGCAAAA TTTACCGGCC 1200
CTATTTCTTC TAGCACTAGC TACTACCTTA TATAATTACT TTAATTTGTA AGTGTCATCA 1260
ATATCATGCA AAATTTAGTC AAAATATTTA TCTCCATGTC TTTGGTTCTC AAATAGAGCA 1320
AATAGACTCA GACTCGAACC TACGCAAGTG TAAAAGCAAG GAATGATTAC CAAACAAGAC 1380
AGTTCTCAAC AAGCAACAAA ATAAACAAGG CAAAACTAGT TAGAAAACGA ATGCTATTGT 1440
CATTCCAGCC GAACTAACAA TAACCTACAT ACAAACCAGT TCAACCTTTA GCTTTACTTT 1500
TACCATTTTT GGCTCTTTTG TTAATTGAGA TTTGAAATAA ATCTCAACAA TAATTTATTT 1560
ATGATCCACA TGACATTAGT CTAAGAGGTG ATTGAACATT ACTTGAGAGA TATTGCTATT 1620
CGATGAGTTA CATAGTTTTC CACTACAAAT TTAATTTACT CTAACTATGA ATATTATAAT 1680
TTGTAGTACA GTTTTTATTT AATAGGTAAA TTTAATAAGA GTAAACAAAA AATATCCAGC 1740
AACTATAGTC TCCAGTCCAA ATTATGTAGA GAAAAGTCTG GAATAACGTC CAAAGCCGCC 1800
CGTCTCTTTT ACTTATAACT GAATTAAATT CTGGATACCA CAGGGTGGAC TATCAATTTT 1860
GTCATAAAAG TCACTGATTC CTCACAACCA CTTGCCTATA AATAGCTTTC ACTTTAGCAT 1920
 
TTGTTTGCCA TCACATTCAA A ATG AGG CGA ACT TCT AAA TTG ACT ACT TTT TCT
Met Arg Arg Thr Ser Lys Leu Thr Thr Phe Ser
1               5                   10

TTG CTG TTT TCT CTG GTT TTG CTG AGT GCT GCC TTG GCA CAG AAT TGT GGT
Leu Leu Phe Ser Leu Val Leu Leu Ser Ala Ala Leu Ala Gln Asn Cys Gly
            15                  20                  25

TCA CAG GGC GGA GGC AAA GTT TGT GCG TCG GGA CAA TGT TGC AGC AAA TTC
Ser Gln Gly Gly Gly Lys Val Cys Ala Ser Gly Gln Cys Cys Ser Lys Phe
    30                  35                  40                  45

GGG TGG TGC GGT AAC ACT AAT GAC CAT TGT GGT TCT GGC AAT TGT CAA AGT
Gly Trp Cys Gly Asn Thr Asn Asp His Cys Gly Ser Gly Asn Cys Gln Ser
                50                  55                  60

CAG TGT CCA GGT GGC GGC CCT GGT CCT GGT CCT GTT ACT GGT GGG GAC CTC
Gln Cys Pro Gly Gly Gly Pro Gly Pro Gly Pro Val Thr Gly Gly Asp Leu
        65                  70                  75

GCA AGC GTC ATC TCA AAT TCT ATG TTT GAT CAA ATG CTT AAG CAT CGT AAC
Ala Ser Val Ile Ser Asn Ser Met Phe Asp Gln Met Leu Lys His Arg Asn
80                  85                  90                  95

GAA AAT TCT TGT CAA GGA AAG AAT AAT TTC TAC AGT TAC AAT GCC TTT ATT
Glu Asn Ser Gys Gln Gly Lys Asn Asn Phe Tyr Ser Tyr Asn Ala Phe Ile
100 105 110

ACT GCT GCT AGG TCT TTT CCT GGC TTT GGT ACA AGT GGT GAT ATC AAT GCC
Thr Ala Ala Arg Ser Phe Pro Gly Phe Gly Thr Ser Gly Asp Ile Asn Ala
115 120 125 130

CGT AAA AGG GAA ATT GCT GCT TTC TTT GCC CAA ACC TCC CAT GAA ACT ACT
Arg Lys Arg Glu Ile Ala Ala Phe Phe Ala Gln Thr Ser His Glu Thr Thr
135 140 145

G GTATGTGTAT AACCATTCAC ATCGAACCAT TAAAATATAA TTTCATTTTA TTTTATTTAG
G

TAATTGATTA TATATGTAG GA GGA TGG CCT TCC GCA CCT GAT GGA CCA TTC GCA
ly Gly Trp Pro Ser Ala Pro Asp Gly Pro Phe Ala
150 155

TGG GGT TAC TGT TTC CTT AGA GAA CGA GGT AAC CCC GGT GAC TAC TGT TCA
Trp Gly Tyr Cys Phe Leu Arg Glu Arg Gly Asn Pro Gly Asp Tyr Cys Ser
160 165 170 175
 
CCA AGT AGT CAA TGG CCT TGT GCA CCT GGA AGG AAA TAT TTC GGA CGA GGC
Pro Ser Ser Gln Trp Pro Cys Ala Pro Gly Arg Lys Tyr Phe Gly Arg Gly
180 185 190

CCA ATC CAA ATT TCA CA GTAAGCTACA TAAATCTATA TATGGTAAAA TTTGATGAAC
Pro Ile Gln Ile Ser Hi
195

TTGTAGTGTC TAATTACGTG TATTTTGACA TTTCAAAACA G C AAC TAC AAC TAT GGG
s Asn Tyr Asn Tyr Gly
200

CCA TGT GGA AGA GCC ATC GGA GTG GAC CTT TTA AAC AAT CCT GAT TTA GTA
Pro Cys Gly Arg Ala Ile Gly Val Asp Leu Leu Asn Asn Pro Asp Leu Val
205 210 215 220

GCC ACA GAC CCA GTC ATC TCA TTC AAG ACT GCT ATC TGG TTC TGG ATG ACC
Ala Thr Asp Pro Val Ile Ser Phe Lys Thr Ala Ile Trp Phe Trp Met Thr
225 230 235

CCT CAA TCA CCA AAG CCT TCT TGC CAC GAT GTC ATC ATT GGA AGA TGG AAC
Pro Gln Ser Pro Lys Pro Ser Cys His Asp Val Ile Ile Gly Arg Trp Asn
240 245 250 255

CCA TCT GCC GGT GAC CGA TCA GCC AAT CGT CTT CCT GGA TTT GGT GTC ATC
Pro Ser Ala Gly Asp Arg Ser Ala Asn Arg Leu Pro Gly Phe Gly Val Ile
260 265 270

ACA AAC ATC ATC AAT GGG GGC CTA GAA TGT GGT CGT GGT AAT GAC AAC AGG
Thr Asn Ile Ile Asn Gly Gly Leu Glu Cys Gly Arg Gly Asn Asp Asn Arg
275 280 285

GTA CAA GAT CGA ATT GGA TTT TAC AGG AGG TAT TGC GGA AGCTT
Val Gln Asp Arg Ile Gly Phe Tyr Arg Arg Tyr Cys Gly
290 295 300 302
**SEQ ID NO : 6**
   TYPE DE SEQUENCE : nucléotidique
   LONGUEUR DE LA SEQUENCE : 71 paires de bases
   NOMBRE DE BRINS : double brin
   ORIGINE : synthétique
   TYPE DE MOLECULE : ADN génonique
   PROPRIETES : oligonucléotide
   CARACTERISTIQUES : 1^{e} paire de base : site BamHI 
GGATCCATGA GGCGAACTTC TAAATTGACT ACTTTTTCTT TGCTGTTTTC TCTGGTTTTG
CTGAGTGCTG C
**SEQ ID NO : 7**
   TYPE DE SEQUENCE : nucléotidique
   LONGUEUR DE LA SEQUENCE : 164 paires de bases
   NOMBRE DE BRINS :
   ORIGINE : synthétique
   TYPE DE MOLECULE : ADN chimérique
   CARACTERISTIQUES : de 1 à 71 paires de bases : séquence de l'ADN génomique de l'endochitinase de tomate
      de 72 à 154 paires de bases : séquence proche de la partie 3' de la séquence de l'ADNc d'endochitinase de tabac 163^{e} paire de bases : site SacI 
GGCCTGGAAT GTGGTCGTGG CAATGACAAT AGGGTCCAGG ATCGCATTGG GTTTTACAGG 60
AGGTATTGCG GTATTCTTGG TGTTAGTCCT GGTGACAATC TTGATTGCGG AAACCAGAGA 120
TCTTTTGGAA ACGGACTTTT AGTCGATACT ATGTAATGAG CTC 164
**SEQ ID NO : 8**
   TYPE DE SEQUENCE : nucléotidique
   LONGUEUR DE LA SEQUENCE : 1863 paires de bases
   NOMBRE DE BRINS : à double brin
   TYPE DE MOLECULE : hybride (ADN génomique - ADN complémentaire)
   PROPRIETES : séquence du gène chimérique complet
   CARACTERISTIQUES : 438^{e} paire de base : ATG d'initiation de la traduction
      1585e paire de base : TAA de terminaison de la traduction
      de 880 à 958 paires de bases : intron 1
      de 1113 à 1193 paires de bases : intron 2. 
AAGCTTGCAC GACACACTTG TCTACTCCAA AAATATCAAA GATACAGTCC TCAGAAGACC 60
AAAGGGCCAA TTGAGACTTT TCAACAAAGG GTAATATCCG GAAACCTCCT CGGATTCCAT 120
TGCCCAGCTA TCTGTCACTT TATTGTGAAG ATAGTGGAAA AGGAAGGTGG CTCCTACAAA 180
TGCCATCATT GCGATAAAGG AAAGGCCATC GTTGAAGATG CCTCTGCCGA CAGTGGTCCC 240
AAAGATGGAC CCCCACCCAC GAGGAGCATC GTGGAAAAAG AAGACGTTCC AACCACGTCT 300
TCAAAGCAAG TGGATTGATG TGATATCTCC ACTGACGTAA GGGATGACGC ACAATCCCAC 360
TATCCTTCGC AAGACCCTTC CTCTATATAA GGAAGTTCAT TTCATTTGGA GAGAACACGG 420
GGGACTCTAG AGGATCCATG AGGCGAACTT CTAAATTGAC TACTTTTTCT TTGCTGTTTT 480
CTCTGGTTTT GCTGAGTGCT GCCTTGGCAC AGAATTGTGG TTCACAGGGC GGAGGCAAAG 540
TTTGTGCGTC GGGACAATGT TGCAGCAAAT TCGGGTGGTG CGGTAACACT AATGACCATT 600
GTGGTTCTGG CAATTGTCAA AGTCAGTGTC CAGGTGGCGG CCCTGGTCCT GGTCCTGTTA 660
CTGGTGGGGA CCTCGGAAGC GTCATCTCAA ATTCTATGTT TGATCAAATG CTTAAGCATC 720
GTAACGAAAA TTCTTGTCAA GGAAAGAATA ATTTCTACAG TTACAATGCC TTTATTACTG 780
CTGCTAGGTC TTTTCCTGGC TTTGGTACAA GTGGTGATAT CAATGCCCGT AAAAGGGAAA 840
TTGCTGCTTT CTTTGCCCAA ACCTCCCATG AAACTACTGG TATGTGTATA ACCATTCACA 900
TCGAACCATT AAAATATAAT TTCATTTTAT TTTATTTAGT AATTGATTAT ATATGTAGGA 960
GGATGGCCTT CCGCACCTGA TGGACCATTC GCATGGGGTT ACTGTTTCCT TAGAGAACGA 1020
GGTAACCCCG GTGACTACTG TTCACCAAGT AGTCAATGGC CTTGTGCACC TGGAAGGAAA 1080
TATTTCGGAC GAGGCCCAAT CCAAATTTCA CAGTAAGCTA CATAAATCTA TATATGGTAA 1140
AATTTGATGA ACTTGTAGTG TCTAATTACG TGTATTTTGA CATTTCAAAA CAGCAACTAC 1200
AACTATGGGC CATGTGGAAG AGCCATCGGA GTGGACCTTT TAAACAATCC TGATTTAGTA 1260
GCCACAGACC CAGTCATCTC ATTCAAGACT GCTATCTGGT TCTGGATGAC CCCTCAATCA 1320
CCAAAGCCTT CTTGCCACGA TGTCATCATT GGAAGATGGA ACCCATCTGC CGGTGACCGA 1380
TCAGCCAATC GTCTTCCTGG ATTTGGTGTC ATCACAAACA TCATCAATGG GGGCCTGGAA 1440
TGTGGTCGTG GCAATGACAA TAGGGTCCAG GATCGCATTG GGTTTTACAG GAGGTATTGC 1500
GGTATTCTTG GTGTTAGTCC TGGTGACAAT CTTGATTGCG GAAACCAGAG ATCTTTTGGA 1560
AACGGACTTT TAGTCGATAC TATGTAATGA GCTCGAATTT CCCCGATCGT TCAAACATTT 1620
GGCAATAAAG TTTCTTAAGA TTGAATCCTG TTGCCGGTCT TGCGATGATT ATCATATAAT 1680
TTCTGTTGAA TTACGTTAAG CATGTAATAA TTAACATGTA ATGCATGACG TTATTTATGA 1740
GATGGGTTTT TATGATTAGA GTCCCGCAAT TATACATTTA ATACGCGATA GAAAACAAAA 1800
TATAGCGCGC AAACTAGGAT AAATTATCGC GCGCGGTGTC ATCTATGTTA CTAGATCGAA 1860
TTC 1863
**SEQ ID NO : 9**
   TYPE DE SEQUENCE : nucléotidique
   LONGUEUR DE LA SEQUENCE : 22 paires de bases
   NOMBRE DE BRINS : simple brin
   ORIGINE : synthétique
   TYPE DE MOLECULE : ADN
   PROPRIETES : sonde (marquée au 32-dcTP) AGGGCCGCCA CCTGGACACT GA
**SEQ ID NO : 10**
   TYPE DE SEQUENCE : acides aminés
   LONGUEUR DE LA SEQUENCE : 20 acides aminés
   PROPRIETES : peptide N-terminal 
Gly Gly Xaa Leu Gly Ser Val Ile Ser Asn Xaa Met Phe Xaa Gln Met
1               5                   10                  15

Leu Lys Xaa Arg
            20
**SEQ ID NO : 11**
   TYPE DE SEQUENCE : nucléotidique
   LONGUEUR DE LA SEQUENCE : 1163 paires de bases
   NOMBRE DE BRINS : à double brin
   TYPE DE MOLECULE : hybride (ADN génomique - ADN complémentaire)
   PROPRIETES : séquence codante du gène chimérique
   CARACTERISTIQUES : 2e paire de bases : site BamHI
      1162° paire de bases : site SacI
      de 7 à 78 paires de bases : séquence 3
      de 79 à 231 paires de bases : séquence 2
      de 232 à 1153 paires de bases : séquence 1
      de 449 à 527 paires de bases : intron 1
      de 682 à 762 paires de bases : intron 2

GGATCC  ATG AGG CGA ACT TCT AAA TTG ACT ACT TTT TCT TTG CTG TTT TCT
        Met Arg Arg Thr Ser Lys Leu Thr Thr Phe Ser Leu Leu Phe Ser
        -75                 -70                 -65

CTG GTT TTG CTG AGT GCT GCC TTG GCA CAG AAT TGT GGT TCA CAG GGC GGA
Leu Val Leu Leu Ser Ala Ala Leu Ala Gln Asn Cys Gly Ser Gln Gly Gly
-60                 -55                 -50                     -45

GGC AAA GTT TGT GCG TCG GGA CAA TGT TGC AGC AAA TTC GGG TGG TGC GGT
Gly Lys Val Cys Ala Ser Gly Gln Cys Cys Ser Lys Phe Gly Trp Cys Gly
            -40                 -35                 -30

AAC ACT AAT GAC CAT TGT GGT TCT GGC AAT TGT CAA AGT CAG TGT CCA GGT
Asn Thr Asn Asp His Cys Gly Ser Gly Asn Cys Gln Ser Gln Cys Pro Gly
    -25                 -20                 -15                 -10

GGC GGC CCT GGT CCT GGT CCT GTT ACT GGT GGG GAC CTC GGA AGC GTC ATC
Gly Gly Pro Gly Pro Gly Pro Val Thr Gly Gly Asp Leu Gly Ser Val Ile
                -5              -1   1               5

TCA AAT TCT ATG TTT GAT CAA ATG CTT AAG CAT CGT AAC GAA AAT TCT TGT
Ser Asn Ser Met Phe Asp Gln Met Leu Lys His Arg Asn Glu Asn Ser Cys
    10                  15                  20                  25

CAA GGA AAG AAT AAT TTC TAC AGT TAC AAT GCC TTT ATT ACT GCT GCT AGG
Gln Gly Lys Asn Asn Phe Tyr Ser Tyr Asn Ala Phe Ile Thr Ala Ala Arg
                30                  35                  40

TCT TTT CCT GGC TTT GGT ACA AGT GGT GAT ATC AAT GCC CGT AAA AGG GAA
Ser Phe Pro Gly Phe Gly Thr Ser Gly Asp Ile Asn Ala Arg Lys Arg Glu
        45                  50                  55

ATT GCT GCT TTC TTT GCC CAA ACC TCC CAT GAA ACT ACT G GTATGTGTAT
Ile Ala Ala Phe Phe Ala Gln Thr Ser His Glu Thr Thr G
 60                  65                  70

AACCATTCAC ATCGAACCAT TAAAATATAA TTTCATTTTA TTTTATTTAG TAATTGATTA

TATATGTAG GA GGA TGG CCT TCC GCA CCT GAT GGA CCA TTC GCA TGG GGT
             ly Gly Trp Pro Ser Ala Pro Asp Gly Pro Phe Ala Trp Gly
                    75                  80                  85

TAC TGT TTC CTT AGA GAA CGA GGT AAC CCC GGT GAC TAC TGT TCA CCA AGT
Tyr Cys Phe Leu Arg Glu Arg Gly Asn Pro Gly Asp Tyr Cys Ser Pro Ser
                90                  95                  100

AGT CAA TGG CCT TGT GCA CCT GGA AGG AAA TAT TTC GGA CGA GGC CCA ATC
Ser Gln Trp Pro Cys Ala Pro Gly Arg Lys Tyr Phe Gly Arg Gly Pro Ile
105 110 115 120

CAA ATT TCA CA GTAAGCTACA TAAATCTATA TATGGTAAAA TTTGATGAAC
Gln Ile Ser Hi

TTGTAGTGTC TAATTACGTG TATTTTGACA TTTCAAAACA G C AAC TAC AAC TAT GGG
s Asn Tyr Asn Tyr Gly
125

CCA TGT GGA AGA GCC ATC GGA GTG GAC CTT TTA AAC AAT CCT GAT TTA GTA
Pro Cys Gly Arg Ala Ile Gly Val Asp Leu Leu Asn Asn Pro Asp Leu Val
130 135 140 145

GCC ACA GAC CCA GTC ATC TCA TTC AAG ACT GCT ATC TGG TTC TGG ATG ACC
Ala Thr Asp Pro Val ILe Ser Phe Lys Thr Ala Ile Trp Phe Trp Met Thr
150 155 160

CCT CAA TCA CCA AAG CCT TCT TGC CAC GAT GTC ATC ATT GGA AGA TGG AAC
Pro Gln Ser Pro Lys Pro Ser Cys His Asp Val Ile Ile Gly Arg Trp Asn
165 170 175 180

CCA TCT GCC GGT GAC CGA TCA GCC AAT CGT CTT CCT GGA TTT GGT GTC ATC
Pro Ser Ala Gly Asp Arg Ser Ala Asn Arg Leu Pro Gly Phe Gly Val Ile
185 190 195

ACA AAC ATC ATC AAT GGG GGC CTG GAA TGT GGT CGT GGC AAT GAC AAT AGG
Thr Asn Ile Ile Asn Gly Gly Leu Glu Cys Gly Arg Gly Asn Asp Asn Arg
200 205 210

GTC CAG GAT CGC ATT GGG TTT TAC AGG AGG TAT TGC GGT ATT CTT GGT GTT
Val Gln Asp Arg Ile Gly Phe Tyr Arg Arg Tyr Cys Gly Ile Leu Gly Val
215 220 225 230

AGT CCT GGT GAC AAT CTT GAT TGC GGA AAC CAG AGA TCT TTT GGA AAC GGA
Ser Pro Gly Asp Asn Leu Asp Cys Gly Asn Gln Arg Ser Phe Gly Asn Gly
235 240 245

CTT TTA GTC GAT ACT ATG TAA TGA GCTC
Leu Leu Val Asp Thr Met End End
250 255

## Revendications

1. Gène recombinant, **caractérisé en ce qu'**il code pour une protéine à activité endochitinase ou pour un précurseur de cette dernière, qui comprend la séquence (1) ci-après:

Gly Gly Asp Leu Gly Ser Val Ile Ser Asn Ser Met Phe Asp Gln Met Leu
Lys His Arg Asn Glu Asn Ser Cys Gln Gly Lys Asn Asn Phe Tyr Ser Tyr
Asn Ala Phe Ile Thr Ala Ala Arg Ser Phe Pro Gly Phe Gly Thr Ser Gly
Asp Ile Asn Ala Arg Lys Arg Glu Ile Ala Ala Phe Phe Ala Gln Thr Ser
His Glu Thr Thr Gly Gly Trp Pro Ser Ala Pro Asp Gly Pro Phe Ala Trp
Gly Tyr Cys Phe Leu Arg Glu Arg Gly Asn Pro Gly Asp Tyr Cys Ser Pro
Ser Ser Gln Trp Pro Cys Ala Pro Gly Arg Lys Tyr Phe Gly Arg Gly Pro
Ile Gln Ile Ser His Asn Tyr Asn Tyr Gly Pro Cys Gly Arg Ala Ile Gly
Val Asp Leu Leu Asn Asn Pro Asp Leu Val Ala Thr Asp Pro Val Ile Ser
Phe Lys Thr Ala Ile Trp Phe Trp Met Thr Pro Gln Ser Pro Lys Pro Ser
Cys His Asp Val Ile Ile Gly Arg Trp Asn Pro Ser Ala Gly Asp Arg Ser
Ala Asn Arg Leu Pro Gly Phe Gly Val Ile Thr Asn Ile Ile Asn Gly Gly
Leu Glu Cys Gly Arg Gly Asn Asp Asn Arg Val Gln Asp Arg Ile Gly Phe
Tyr Arg Arg Tyr Cys Gly Ile Leu Gly Val Ser Pro Gly Asp Asn Leu Asp
Cys Gly Asn Gln Arg Ser Phe Gly Asn Gly Leu Leu Val Asp Thr Met .

2. Gène recombinant selon la revendication 1, **caractérisé en ce qu'**il code pour une protéine qui comprend, immédiatement en amont de la séquence (1), la séquence (2) ci-après :

Gln Asn Cys Gly Ser Gln Gly Gly Gly Lys Val Cys Ala Ser Gly Gln Cys
Cys Ser Lys Phe Gly Trp Cys Gly Asn Thr Asn Asp His Cys Gly Ser Gly
Asn Cys Gln Ser Gln Cys Pro Gly Gly Gly Pro Gly Pro Gly Pro Val Thr

3. Gène recombinant selon la revendication 1 ou 2, **caractérisé en ce qu'**il code pour une protéine dont la séquence comprend, en amont de la séquence (1), une séquence codant pour un peptide-signal.

4. Gène recombinant selon la revendication 2, **caractérisé en ce qu'**il code pour une protéine dont la séquence comprend, immédiatement en amont de la séquence (2), la séquence (3) ci-après :

Met Arg Arg Thr Ser Lys Leu Thr Thr Phe Ser Leu Leu Phe Ser Leu Val
Leu Leu Ser Ala Ala Leu Ala

5. Gène recombinant selon l'une des revendications 1 à 4, **caractérisé en ce que** sa partie codante comprend au moins une partie 5' de l'ADN génomique de l'endochitinase de tomate et au moins une partie 3' de l'ADN complémentaire de l'endochitinase de tabac.

6. Gène recombinant selon la revendication 5, **caractérisé en ce que** la partie codante comprend au moins un intron.

7. Gène recombinant selon l'une quelconque des revendications 1 à 6, **caractérisé en ce que** sa partie codante est la séquence ci-après :

ATGAGGCGAA CTTCTAAATT GACTACTTTT TCTTTGCTGT TTTCTCTGGT TTTGCTGAGT
GCTGCCTTGG CACAGAATTG TGGTTCACAG GGCGGAGGCA AAGTTTGTGC GTCGGACAA
TGTTGCAGCA AATTCGGGTG GTGCGGTAAC ACTAATGACC ATTGTGGTTC TGGCAATTGT
CAAAGTCAGT GTCCAGGTGG CGGCCCTGGT CCTGGTCCTG TTACTGGTGG GGACCTCGGA
AGCGTCATCT CAAATTCTAT GTTTGATCAA ATGCTTAAGC ATCGTAACGA AAATTCTTGT
CAAGGAAAGA ATAATTTCTA CAGTTACAAT GCCTTTATTA CTGCTGCTAG GTCTTTTCCT
GGCTTTGGTA CAAGTGGTGA TATCAATGCC CGTAAAAGGG AAATTGCTGC TTTCTTTGCC
CAAACCTCCC ATGAAACTAC TGGTATGTGT ATAACCATTC ACATCGAACC ATTAAAATAT
AATTTCATTT TATTTTATTT AGTAATTGAT TATATATGTA GGAGGATGGC CTTCCGCACC
TGATGGACCA TTCGCATGGG GTTACTGTTT CCTTAGAGAA CGAGGTAACC CCGGTGACTA
CTGTTCACCA AGTAGTCAAT GGCCTTGTGC ACCTGGAAGG AAATATTTCG GACGAGGCCC
AATCCAAATT TCACAGTAAG CTACATAAAT CTATATATGG TAAAATTTGA TGAACTTGTA
GTGTCTAATT ACGTGTATTT TGACATTTCA AAACAGCAAC TACAACTATG GGCCATGTGG
AAGAGCCATC GGAGTGGACC TTTTAAACAA TCCTGATTTA GTAGCCACAG ACCCAGTCAT
CTCATTCAAG ACTGCTATCT GGTTCTGGAT GACCCCTCAA TCACCAAAGC CTTCTTGCCA
CGATGTCATC ATTGGAAGAT GGAACCCATC TGCCGGTGAC CGATCAGCCA ATCGTCTTCC
TGGATTTGGT GTCATCACAA ACATCATCAA TGGGGGCCTG GAATGTGGTC GTGGCAATGA
CAATAGGGTC CAGGATCGCA TTGGGTTTTA CAGGAGGTAT TGCGGTATTC TTGGTGTTAG
TCCTGGTGAC AATCTTGATT GCGGAAACCA GAGATCTTTT GGAAACGGAC TTTTAGTCGA
TACTATGTAA TGA

8. Gène recombinant selon l'une des revendications 1 à 7, **caractérisé en ce qu'**il comprend une séquence promotrice comportant le promoteur 35S du virus de la mosaïque de chou-fleur.

9. Gène recombinant selon l'une des revendications 1 à 8, **caractérisé en ce qu'**il comprend une séquence terminatrice comportant le terminateur de la nopaline synthase d'Agrobacterium tumefaciens.

10. Protéine à activité endochitinase, **caractérisée en ce qu'**elle comprend la séquence (1).

11. Cellule végétale, **caractérisée en ce qu'**elle est transformée par un gène recombinant selon l'une des revendications 1 à 9.

12. Cellule végétale selon la revendication 11, **caractérisée en ce qu'**elle appartient à l'une des espèces Nicotiana tabacum, Helianthus annuus et Brassica napus.

13. Plante ou partie de plante, **caractérisée en ce qu'**elle contient, dans un contexte susceptible de permettre son expression, un gène recombinant selon l'une des revendications 1 à 9.

14. Plante ou partie de plante selon la revendication 13, **caractérisée en ce qu'**elle appartient à l'une des espèces Nicotiana tabacum, Helianthus annuus et Brassica napus.

15. Partie de plante selon la revendication 13 ou 14, **caractérisée en ce qu'**elle est une semence.

16. Procédé pour obtenir des plantes résistantes aux agents pathogènes, **caractérisé en ce qu'**il comprend une étape de transformation de cellules végétales par un gène recombinant selon l'une des revendications 1 à 9, suivie d'une étape de multiplication des cellules transformées et d'une étape de régénération des plantes.

17. Procédé selon la revendication 16, **caractérisé en ce qu'**il est appliqué à l'une des espèces Nicotiana tabacum, Helianthus annuus et Brassica napus.

18. Procédé pour obtenir la protéine selon la revendication 10, **caractérisé en ce qu'**il comprend la mise en culture de cellules ou de cals végétaux contenant, dans un contexte susceptible de permettre son expression, un gène recombinant selon l'une des revendications 1 à 9, la lyse de ceux-ci, l'isolement et la purification de cette protéine.

19. Procédé selon la revendication 18, **caractérisé en ce que** les cellules ou les cals végétaux sont choisis dans l'une des espèces Nicotiana tabacum, Helianthus annuus et Brassica napus.

## Patentansprüche

1. Rekombinantes Gen, **dadurch gekennzeichnet, dass** es für ein Protein mit Endochitinase-Aktivität oder einen Vorläufer desselben codiert, das bzw. der die nachstehende Sequenz (1) aufweist:

Gly Gly Asp Leu Gly Ser Val Ile Ser Asn Ser Met Phe Asp Gln Met Leu
Lys His Arg Asn Glu Asn Ser Cys Gln Gly Lys Asn Asn Phe Tyr Ser Tyr
Asn Ala Phe Ile Thr Ala Ala Arg Ser Phe Pro Gly Phe Gly Thr Ser Gly
Asp Ile Asn Ala Arg Lys Arg Glu Ile Ala Ala Phe Phe Ala Gln Thr Ser
His Glu Thr Thr Gly Gly Trp Pro Ser Ala Pro Asp Gly Pro Phe Ala Trp
Gly Tyr Cys Phe Leu Arg Glu Arg Gly Asn Pro Gly Asp Tyr Cys Ser Pro
Ser Ser Gln Trp Pro Cys Ala Pro Gly Arg Lys Tyr Phe Gly Arg Gly Pro
Ile Gln Ile Ser His Asn Tyr Asn Tyr Gly Pro Cys Gly Arg Ala Ile Gly
Val Asp Leu Leu Asn Asn Pro Asp Leu Val Ala Thr Asp Pro Val Ile Ser
Phe Lys Thr Ala Ile Trp Phe Trp Met Thr Pro Gln Ser Pro Lys Pro Ser
Cys His Asp Val Ile Ile Gly Arg Trp Asn Pro Ser Ala Gly Asp Arg Ser
Ala Asn Arg Leu Pro Gly Phe Gly Val Ile Thr Asn Ile Ile Asn Gly Gly
Leu Glu Cys Gly Arg Gly Asn Asp Asn Arg Val Gln Asp Arg Ile Gly Phe
Tyr Arg Arg Tyr Cys Gly Ile Leu Gly Val Ser Pro Gly Asp Asn Leu Asp
Cys Gly Asn Gln Arg Ser Phe Gly Asn Gly Leu Leu Val Asp Thr Met

2. Rekombinantes Gen nach Anspruch 1, **dadurch gekennzeichnet, dass** es für ein Protein codiert, das unmittelbar stromaufwärts der Sequenz (1) die nachstehende Sequenz (2) aufweist:

Gln Asn Cys Gly Ser Gln Gly Gly Gly Lys Val Cys Ala Ser Gly Gln Cys Cys
Ser Lys Phe Gly Trp Cys Gly Asn Thr Asn Asp His Cys Gly Ser Gly
Asn Cys Gln Ser Gln Cys Pro Gly Gly Gly Pro Gly Pro Gly Pro Val Thr

3. Rekombinantes Gen nach Anspruch 1 oder 2, **dadurch gekennzeichnet, dass** es für ein Protein codiert, dessen Sequenz stromaufwärts der Sequenz (1) eine für ein Peptidsignal codierende Sequenz aufweist.

4. Rekombinantes Gen nach Anspruch 2, **dadurch gekennzeichnet, dass** es für ein Protein codiert, dessen Sequenz unmittelbar stromaufwärts der Sequenz (2) die nachstehende Sequenz (3) aufweist:

Met Arg Arg Thr Ser Lys Leu Thr Thr Phe Ser Leu Leu Phe Ser Leu Val
Leu Leu Ser Ala Ala Leu Ala

5. Rekombmantes Gen nach einem der Ansprüche 1 bis 4, **dadurch gekennzeichnet, dass** sein codierender Teil mindestens einen 5'-Teil der genomischen Endochitinase-DNA der Tomate und mindestens einen 3'-Teil der komplementären Endochitinase-DNA von Tabak aufweist.

6. Rekombinantes Gen nach Anspruch 5, **dadurch gekennzeichnet, dass** der codierende Teil mindestens ein Intron aufweist.

7. Rckombinantes Gen nach einem der Ansprüche 1 bis 6, **dadurch gekennzeichnet, dass** sein codierender Teil die nachstehende Sequenz ist:

ATGAGGCGAA CTTCTAAATT GACTACTTTT TCTTTGCTGT TTTCTCTGGT TTTGCTGAGT
GCTGCCTTGG CACAGAATTG TGGTTCACAG GGCGGAGGCA AAGTTTGTGC GTCGGACAA
TGTTGCAGCA AATTCGGGTG GTGCGGTAAC ACTAATGACC ATTGTGGTTC TGGCAATTGT
CAAAGTCAGT GTCCAGGTGG CGGCCCTGGT CCTGGTCCTG TTACTGGTGG GGACCTCGGA
AGCGTCATCT CAAATTCTAT GTTTGATCAA ATGCTTAAGC ATCGTAACGA AAATTCTTGT
CAAGGAAAGA ATAATTTCTA CAGTTACAAT GCCTTTATTA CTGCTGCTAG GTCTTTTCCT
GGCTTTGGTA CAAGTGGTGA TATCAATGCC CGTAAAAGGG AAATTGCTGC TTTCTTTGCC
CAAACCTCCC ATGAAACTAC TGGTATGTGT ATAACCATTC ACATCGAACC ATTAAAATAT
AATTTCATTT TATTTTATTT AGTAATTGAT TATATATGTA GGAGGATGGC CTTCCGCACC
TGATGGACCA TTCGCATGGG GTTACTGTTT CCTTAGAGAA CGAGGTAACC CCGGTGACTA
CTGTTCACCA AGTAGTCAAT GGCCTTGTGC ACCTGGAAGG AAATATTTCG GACGAGGCCC
AATCCAAATT TCACAGTAAG CTACATAAAT CTATATATGG TAAAATTTGA TGAACTTGTA
GTGTCTAATT ACGTGTATTT TGACATTTCA AAACAGCAAC TACAACTATG GGCCATGTGG
AAGAGCCATC GGAGTGGACC TTTTAAACAA TCCTGATTTA GTAGCCACAG ACCCAGTCAT
CTCATTCAAG ACTGCTATCT GGTTCTGGAT GACCCCTCAA TCACCAAAGC CTTCTTGCCA
CGATGTCATC ATTGGAAGAT GGAACCCATC TGCCGGTGAC CGATCAGCCA ATCGTCTTCC
TGGATTTGGT GTCATCACAA ACATCATCAA TGGGGGCCTG GAATGTGGTC GTGGCAATGA
CAATAGGGTC CAGGATCGCA TTGGGTTTTA CAGGAGGTAT TGCGGTATTC TTGGTGTTAG
TCCTGGTGAC AATCTTGATT GCGGAAACCA GAGATCTTTT GGAAACGGAC TTTTAGTCGA
TACTATGTAA TGA

8. Rekombinantes Gen nach einem der Ansprüche 1 bis 7, **dadurch gekennzeichnet, dass** es eine Promotor-Sequenz mit dem Promotor 35S des Blumenkohl-Mosaikvirus aufweist.

9. Rekombinantes Gen nach einem der Ansprüche 1 bis 8, **dadurch gekennzeichnet, dass** es eine Terminationssequenz mit dem Terminator der Nopalinsynthase von Agrobacterium tumefaciens aufweist.

10. Protein mit Endochitinasc-Aktivität, **dadurch gekennzeichnet, dass** es die Sequenz (1) aufweist.

11. Pflanzliche Zelle, **dadurch gekennzeichnet, dass** sie durch ein rekombinantes Gen nach einem der Ansprüche 1 bis 9 transformiert ist.

12. Pflanzliche Zelle nach Anspruch 11, **dadurch gekennzeichnet, dass** sie zu einer der folgenden Arten gehört: Nicotiana tabacum, Helianthus annuus und Brassica napus.

13. Pflanze oder Pflanzenteil, **dadurch gekennzeichnet, dass** sie bzw. er ein rekombinantes Gen nach einem der Ansprüche 1 bis 9 in einem Kontext enthält, der die Expression desselben ermöglichen kann.

14. Pflanze oder Pflanzenteil nach Anspruch 13, **dadurch gekennzeichnet, dass** sie bzw. er zu einem der folgenden Arten gehört: Nicotiana tabacum, Helianthus annuus und Brassica napus.

15. Pflanzenteil nach Anspruch 13 oder 14, **dadurch gekennzeichnet, dass** er ein Samen ist.

16. Verfahren zur Herstellung von Pflanzen, die gegen Pathogene resistent sind, **dadurch gekennzeichnet, dass** es einen Schritt der Transformation von pflanzlichen Zellen durch ein rekombinantes Gen nach einem der Ansprüche 1 bis 9, gefolgt von einem Schritt der Multiplikation der transformierten Zellen und einem Schritt der Regeneration der Pflanzen umfasst.

17. Verfahren nach Anspruch 16, **dadurch gekennzeichnet, dass** es bei einer der folgenden Arten angewendet wird: Nicotiana tabacum, Helianthus annuus und Brassica napus.

18. Verfahren zur Herstellung des Proteins nach Anspruch 10, **dadurch gekennzeichnet, dass** es die Kultivierung von pflanzlichen Zellen oder pflanzlichem Kallus, die ein rekombinantes Gen nach einem der Ansprüche 1 bis 9 in einem Kontext enthalten, der die Expression desselben ermöglichen kann, die Lyse derselben, die Isolierung und die Reinigung dieses Proteins umfasst.

19. Verfahren nach Anspruch 18, **dadurch gekennzeichnet, dass** die pflanzlichen Zellen oder der pflanzliche Kallus unter den folgenden Arten ausgewählt sind: Nicotiana tabacum, Helianthus annuus und Brassica napus.

## Claims

1. A recombinant gene **characterized in that** it encodes a protein having endochitinase activity or a precursor thereof, said protein comprising the sequence (1) below :

Gly Gly Asp Leu Gly Ser Val Ile Ser Asn Ser Met Phe Asp Gln Met
Leu Lys His Arg Asn Glu Asn Ser Cys Gln Gly Lys Asn Asn Phe Tyr
Ser Tyr Asn Ala Phe Ile Thr Ala Ala Arg Ser Phe Pro Gly Phe Gly
Thr Ser Gly Asp Ile Asn Ala Arg Lys Arg Glu Ile Ala Ala Phe Phe
Ala Gln Thr Ser His Glu Thr Thr Gly Gly Trp Pro Ser Ala Pro Asp
Gly Pro Phe Ala Trp Gly Tyr Cys Phe Leu Arg Glu Arg Gly Asn Pro
Gly Asp Tyr Cys Ser Pro Ser Ser Gln Trp Pro Cys Ala Pro Gly Arg
Lys Tyr Phe Gly Arg Gly Pro Ile Gln Ile Ser His Asn Tyr Asn Tyr
Gly Pro Cys Gly Arg Ala Ile Gly Val Asp Leu Leu Asn Asn Pro Asp
Leu Val Ala Thr Asp Pro Val Ile Ser Phe Lys Thr Ala Ile Trp Phe
Trp Met Thr Pro Gln Ser Pro Lys Pro Ser Cys His Asp Val Ile Ile
Gly Arg Trp Asn Pro Ser Ala Gly Asp Arg Ser Ala Asn Arg Leu Pro
Gly Phe Gly Val Ile Thr Asn Ile Ile Asn Gly Gly Leu Glu Cys Gly
Arg Gly Asn Asp Asn Arg Val Gln Asp Arg Ile Gly Phe Tyr Arg Arg
Tyr Cys Gly Ile Leu Gly Val Ser Pro Gly Asp Asn Leu Asp Cys Gly
Asn Gln Arg Ser Phe Gly Asn Gly Leu Leu Val Asp Thr Met

2. The recombinant gene of claim 1, **characterized in that** it encodes a protein which comprises, immediately upstream of the sequence (1), the sequence (2) below :

Gln Asn Cys Gly Ser Gln Gly Gly Gly Lys Val Cys Ala Ser Gly Gln
Cys Cys Ser Lys Phe Gly Trp Cys Gly Asn Thr Asn Asp His Cys Gly
Ser Gly Asn Cys Gln Ser Gln Cys Pro Gly Gly Gly Pro Gly Pro Gly
Pro Val Thr

3. The recombinant gene of claim 1 or 2, **characterized in that** it encodes a protein whose sequence comprises, upstream of the sequence (1), a sequence coding for a signal peptide.

4. The recombinant gene of claim 2, **characterized in that** it encodes a protein whose sequence comprises, immediately upstream of the sequence (2), the sequence (3) below :

Met Arg Arg Thr Ser Lys Leu Thr Thr Phe Ser Leu Leu Phe Ser Leu
Val Leu Leu Ser Ala Ala Leu Ala

5. The recombinant gene of one of claims 1 to 4, **characterized in that** its coding portion comprises at least one 5' portion of the genomic DNA of tomato endochitinase and at least one 3' portion of the complementary DNA of tobacco endochitinase.

6. The recombinant gene of claim 5, **characterized in that** the coding portion comprises at least one intron.

7. The recombinant gene of one of claims 1 to 6, **characterized in that** its coding portion is represented by the sequence below :

ATGAGGCGAA CTTCTAAATT GACTACTTTT TCTTTGCTGT TTTCTCTGGT TTTGCTGAGT
GCTGCCTTGG CACAGAATTG TGGTTCACAG GGCGGAGGCA AAGTTTGTGC GTCGGGACAA
TGTTGCAGCA AATTCGGGTG GTGCGGTAAC ACTAATGACC ATTGTGGTTC TGGCAATTGT
CAAAGTCAGT GTCCAGGTGG CGGCCCTGGT CCTGGTCCTG TTACTGGTGG GGACCTCGGA
AGCGTCATCT CAAATTCTAT GTTTGATCAA ATGCTTAAGC ATCGTAACGA AAATTCTTGT
CAAGGAAAGA ATAATTTCTA CAGTTACAAT GCCTTTATTA CTGCTGCTAG GTCTTTTCCT
GGCTTTGGTA CAAGTGGTGA TATCAATGCC CGTAAAAGGG AAATTGCTGC TTTCTTTGCC
CAAACCTCCC ATGAAACTAC TGGTATGTGT ATAACCATTC ACATCGAACC ATTAAAATAT
AATTTCATTT TATTTTATTT AGTAATTGAT TATATATGTA GGAGGATGGC CTTCCGCACC
TGATGGACCA TTCGCATGGG GTTACTGTTT CCTTAGAGAA CGAGGTAACC CCGGTGACTA
CTGTTCACCA AGTAGTCAAT GGCCTTGTGC ACCTGGAAGG AAATATTTCG GACGAGGCCC
AATCCAAATT TCACAGTAAG CTACATAAAT CTATATATGG TAAAATTTGA TGAACTTGTA
GTGTCTAATT ACGTGTATTT TGACATTTCA AAACAGCAAC TACAACTATG GGCCATGTGG
AAGAGCCATC GGAGTGGACC TTTTAAACAA TCCTGATTTA GTAGCCACAG ACCCAGTCAT
CTCATTCAAG ACTGCTATCT GGTTCTGGAT GACCCCTCAA TCACCAAAGC CTTCTTGCCA
CGATGTCATC ATTGGAAGAT GGAACCCATC TGCCGGTGAC CGATCAGCCA ATCGTCTTCC
TGGATTTGGT GTCATCACAA ACATCATCAA TGGGGGCCTG GAATGTGGTC GTGGCAATGA
CAATAGGGTC CAGGATCGCA TTGGGTTTTA CAGGAGGTAT TGCGGTATTC TTGGTGTTAG
TCCTGGTGAC AATCTTGATT GCGGAAACCA GAGATCTTTT GGAAACGGAC TTTTAGTCGA
TACTATGTAA TGA

8. The recombinant gene of one of claims 1 to 7, **characterized in that** it comprises a promoter sequence containing the 35S promoter of cauliflower mosaic virus.

9. The recombinant gene of one of claims 1 to 8, **characterized in that** it comprises a termination sequence containing the nopaline synthese terminator of *Agrobacterium tumefaciens.*

10. A protein having endochitinase activity, **characterized in that** it comprises the sequence (1).

11. A plant cell, **characterized in that** it is transformed by a recombinant gene according to one of claims 1 to 9.

12. The plant cell of claim 11, **characterized in that** it belongs to one of the species *Nicotiana tabacum, Helianthus annuus* or *Brassica napus.*

13. A plant or plant part, **characterized in that** it contains, in a context capable of permitting its expression, a recombinant gene according to one of claims 1 to 9.

14. The plant or plant part of claim 13 **characterized in that** it belongs to one of the species *Nicotiana tabacum, Helianthus annuus* or *Brassica napus.*

15. The plant part of claim 13 or 14, **characterized in that** it is a seed.

16. A method of obtaining plants resistant to pathogenic agents, **characterized in that** it comprises a step of transformation of plant cells by a recombinant gene according to one of claims 1 to 9, followed by a step of multiplication of the transformed cells and a step of regeneration of the plants.

17. The method of claim 16, **characterized in that** it is applied to one of the species *Nicotiana tabacum, Helianthus annuus* or *Brassica napus.*

18. A method of obtaining the protein of claim 10, **characterized in that** it comprises the culturing of plant cells or calluses containing, in a context capable of permitting its expression, a recombinant gene according to one of claims 1 to 9, lysis of these cells or calluses, and isolation and purification of this protein.

19. The method of claim 18, **characterized in that** the plant cells or calluses are selected from one of the species *Nicotania tabacum, Helianthus annuus* or *Brassica napus.*
